# EUROPEAN PATENT APPLICATION

(11) **EP 4 799 678 A1**
(43) Date of publication of application: **02.09.2026**
(21) Application number: 26159659.7
(22) Date of filing: 19.02.2026
(51) Int. Cl.: A61N 1/372, A61N 1/375

(54) **MULTI-CHAMBER LEADLESS PACEMAKER SYSTEM WITH DRIFT COMPENSATION**

(30) Priority: 26.02.2025 US 202563763841 P; 12.02.2026 US 202619538718; 12.02.2026 US 202619538724
(71) Applicant: Pacesetter, Inc., Sylmar, CA 91342 (US)
(72) Inventor: Xin, Xiyao, Northridge, CA (US); Sison, Shiloh, Alameda, CA (US); Chen, Xi Lin, Stevenson Ranch, CA (US)
(74) Representative: Barker Brettell Sweden AB

(57) **Abstract**

Leadless pacemakers (LPs) and systems for use therewith are disclosed. During a first period of time, during which an LP (102a, 102b) receives event messages from a second LP (102b, 102a), the LP (102a, 102b) times its pacing of a first cardiac chamber based on the event messages received from the second LP (102b, 102a) so that AV synchrony is maintained, and the LP (102a, 102b) determines and stores count value(s). Based on at least one of the count value(s), the LP (102a, 102b) determines a compensation offset indicative of a drift between timing circuitry (160) of the LP (102a, 102b) and timing circuitry (160) of the second LP (102b, 102a). During a second period of time, during which the LP (102a, 102b) does not receive event messages from the second LP (102b, 102a), the LP (102a, 102b) times its pacing of the first cardiac chamber based on the compensation offset to thereby compensate for the drift so that the AV synchrony is also maintained during the second period of time.

## Description

### FIELD OF TECHNOLOGY

Embodiments described herein generally relate to devices, and systems for providing drift compensation in a multi-chamber leadless pacemaker system that includes at least two leadless pacemakers.

### BACKGROUND

An example of a multi-chamber leadless pacemaker (LP) system is a dual-chamber LP system including an atrial leadless pacemaker (aLP) and a ventricular leadless pacemaker (vLP) that utilize implant-to-implant (i2i) communication to coordinate their dual-chamber functionality. More specifically, such a multi-chamber LP system may utilize an i2i communication protocol that requires the aLP to transmit an i2i event message to the vLP whenever the aLP senses an intrinsic atrial event or causes a paced atrial event. Similarly, the i2i communication protocol may also require the vLP to transmit an i2i event message to the aLP whenever the vLP senses an intrinsic ventricular event or causes a paced ventricular event. The i2i event messages sent between the LPs can be conductive communication messages. That is, conductive communication, which is more energy efficient than radio frequency (RF) communication and inductive communication, may be utilized for the i2i communication. Alternatively, RF or inductive communication may be utilized for the i2i communication that takes place between LPs.

In such a dual chamber LP system, the vLP may time its delivery of ventricular pacing stimulation (to the ventricular chamber in or on which the vLP is implanted) such that the ventricular pacing stimulation is delivered at a specified atrioventricular interval (AVI) after an atrial pacing stimulation was delivered by the aLP. Similarly, the aLP may time its delivery of atrial pacing stimulation (to the atrial chamber in or one which the aLP is implanted) such that the atrial pacing stimulation is delivered at a specified ventricular-atrial interval (VAI) is delivered after a ventricular pacing stimulation was delivered by the vLP. Such operation of the dual chamber LP system may occur, e.g., when the dual chamber LP system is in a DOO mode. However, where electromagnetic interference (EMI) and/or other noise is present, which prevents aLP and the vLP from successfully performing i2i communication, the dual chamber LP system may revert to a VOO mode during which only the vLP delivers pacing stimulation. In other words, EMI and/or other noise may cause a dual chamber LP system that is operating in a dual chamber pacing mode, such as the DOO mode, to transition to a signal chamber pacing mode, such as the VOO mode. It is also possible that such a dual chamber LP system may purposely transition from operating in the DOO mode to operating in an AAI+VVI mode during which the aLP and the vLP purposely abstain from transmitting event messages to one another to conserve power.

### SUMMARY

An aspect of the present technology is related to a leadless pacemaker (LP) configured to communicate with a second LP using implant-to-implant (i2i) communication. The LP is configured to perform pacing of a first cardiac chamber, and the second LP is configured to perform pacing of a second cardiac chamber. The LP comprises a receiver configured to receive event messages from the second LP using i2i communication, a plurality of electrodes, a memory, a timing circuitry, a pulse generator configured to deliver pacing pulses using at least two of the plurality of electrodes, and a controller communicatively coupled to the receiver, the pulse generator, and the memory. The controller is configured to. during a first period of time, during which event messages are received by the receiver from the second LP using i2i communication, control the pulse generator to time pacing of the first cardiac chamber based on the event messages received from the second LP using i2i communication so that atrioventricular (AV) synchrony is maintained during the first period of time. The controller is also configured to, during the first period of time, during which the event messages are received by the receiver from the second LP using i2i communication, determine and store, in the memory, one or more count values produced using one or more timers of the timing circuitry of the LP. The controller is further configured to determine a compensation offset based on at least one of the one or more count values determined and stored during the first period of time. The compensation offset is indicative of a drift between the timing circuitry of the LP and timing circuitry of the second LP. The controller is also configured to, during a second period of time, during which event messages are not received by the receiver from the second LP, control the pulse generator to time pacing of the first cardiac chamber based on the compensation offset, to thereby compensate for the drift between the timing circuitry of the LP and the timing circuitry of the second LP so that the AV synchrony is maintained during the second period of time.

In an embodiment, the controller is configured to determine and store, in the memory and during the first period of time, a measured count value corresponding to a duration between when the controller controls the pulse generator to deliver pacing stimulation to the first cardiac chamber and when the receiver receives an event message, from the second LP, informing LP that the second LP delivered or is about to deliver pacing stimulation to the second cardiac chamber. The controller is also configured, in this embodiment, to determine the compensation offset by subtracting the measured count value from an expected count value. The expected count value corresponds to what the measured count value would be if there was no drift between the timing circuitry of the LP and the timing circuitry of the second LP.

In an embodiment, the timing circuitry of the LP comprises a high-resolution counter and a low-resolution counter. The controller is, in this embodiment, configured to use the high-resolution counter to determine, during the first period of time, and store in the memory, an expected high-resolution count value (E_high_res) and a measured high-resolution count value (M_high_res). The controller is also configured to determine as the compensation offset the expected high-resolution count value (E_high_res) minus the measured high-resolution count value (M_high_res).

In an embodiment, the controller is configured to use the low-resolution counter to determine, during the first period of time, and store in the memory, a calculated low-resolution count value (C_low_res) and a remaining low-resolution count value (R_low_res). The controller is also configured to control the pulse generator, during the second period of time, to deliver further pacing stimulation to the first cardiac chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order.

In an embodiment, the LP further comprises a low-resolution counter and a high-resolution counter. During the first period of time, the controller is configured to determine and store, in the memory, each of the following: a beginning low-resolution count value (B_low_res) of the low-resolution counter and a beginning high-resolution count value (B_high_res) of the high-resolution counter corresponding to when the controller controls the pulse generator to deliver pacing stimulation to the first cardiac chamber; a measured low-resolution count value (M_low_res) of the low-resolution counter and a measured high-resolution count value (M_high_res) of the high-resolution counter corresponding to when the receiver receives one of the event messages, from the second LP, informing the LP that the second LP delivered or is about to deliver pacing stimulation to the second cardiac chamber; a remaining low-resolution count value (R_low_res) corresponding to a base rate low-resolution count value (BR_low_res) minus the measured low-resolution count value (M_low_res); and an expected high-resolution count value (E_high_res) determined based on a sum of the beginning high-resolution count value (B_high_res) and a calculated high-resolution count value (C_high_res). The base rate low-resolution count value (BR_low_res) corresponds to how many counts of the low-resolution counter occur during a base pacing rate interval. The calculated high-resolution count value (C_high_res) is calculated based on the programmed AV interval. The controller is also configured to determine as the compensation offset the expected high-resolution count value (E_high_res) minus the measured high-resolution count value (M_high_res).

In an embodiment, to calculate the calculated high-resolution count value (C_high_res) based on the programmed AV interval, if the LP is configured to be implanted in or on a ventricular chamber or proximate a left bundle branch (LBB) and to perform pacing of the ventricular chamber, then the calculated high-resolution count value (C_high_res) corresponds to the base pacing rate interval minus a programmed AV interval. Alternatively, if the LP is configured to be implanted in or on an atrial chamber and to perform pacing of the atrial chamber, then the calculated high-resolution count value (C_high_res) corresponds to the programmed AV interval.

In an embodiment, the high-resolution counter comprises a modulo-n counter that is configured to count from zero to n-1 and then reset back to zero. The controller is configured, in this embodiment, to determine that the expected high-resolution count value (E_high_res) is equal to a modulo-n operation on the sum of the beginning high-resolution count value (B_high_res) and the calculated high-resolution count value (C_high_res).

In an embodiment, in order to time pacing of the first cardiac chamber during the second period of time, based on the compensation offset that the LP determined and stored, the controller is configured to starting when the controller most recently controlled the pulse generator to deliver pacing stimulation to the first cardiac chamber, serially one after another in any order, and use the low-resolution counter to count to the remaining low-resolution count value (R_low_res). The calculated low-resolution count value (C_low_res) is equal to the measured low-resolution count value (M_low_res) minus the beginning low-resolution count value (B_low_res). Use of the low-resolution counter to count to the measured low-resolution count value (M_low_res) and to count to the remaining low-resolution count value (R_low_res) is optionally performed by using the low-resolution counter to count to a sum of the measured low-resolution count value (M_low_res) and the remaining low-resolution count value (R_low_res). The controller is also configured to control the pulse generator to deliver further pacing stimulation to the first cardiac chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order..In an embodiment, in order to time pacing of the first cardiac chamber during the second period of time, based on the compensation offset that the LP determined and stored, the controller is configured to, starting when the LP most recently delivered pacing stimulation to the first cardiac chamber, serially one after another in any order, use the low-resolution counter to count to a calculated low-resolution count value (C_low_res), use the high-resolution counter to count to the compensation offset, and use the low-resolution counter to count to the remaining low-resolution count value (R_low_res). Use of the low-resolution counter to count to the calculated low-resolution count value (C_low_res) and to count to the remaining low-resolution count value (R_low_res) is optionally performed by using the low-resolution counter to count to a sum of the calculated low-resolution count value (C_low_res) and the remaining low-resolution count value (R_low_res). The controller is also configured to control the pulse generator to deliver further pacing stimulation to the first cardiac chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order. In this embodiment, the calculated low-resolution count value (C_low_res) is equal to the measured low-resolution count value (M_low_res) minus the beginning low-resolution count value (B_low_res).

In an embodiment, the controller is configured to determine a plurality of the compensation offsets based on the count values determined during the first period of time. The controller is also configured to determine an average of the plurality of compensation offsets, and use the average as the compensation offset during the second period of time to compensate for the drift between the timing circuitry of the LP and the timing circuitry of the second LP so that the AV synchrony is maintained during the second period of time.

In an embodiment, the LP is configured to be implanted in or on a ventricular chamber or proximate the LBB and to perform pacing of the ventricular chamber; and the second LP is configured to be implanted in or on an atrial chamber and to perform pacing of the atrial chamber. In certain such embodiments, to time its pacing of the first cardiac chamber during the second period of time, based on the compensation offset determined and stored during the first period of time, the controller is configured to determine an expected time at which the second LP delivers pacing stimulation to the second cardiac chamber, and at a compensated AV interval, following the expected time at which the second LP delivers pacing stimulation to the second cardiac chamber, control the pulse generator to deliver pacing stimulation to the first cardiac chamber. The compensated AV interval is based on the compensation offset and a programmed AV interval. In certain such embodiments, the compensated AV interval is equal to the programmed AV interval minus the compensation offset.

In an embodiment, the LP is configured to be implanted in or on an atrial chamber and to perform pacing of the atrial chamber, and the second LP is configured to be implanted in or on a ventricular chamber or proximate the LBB and to perform pacing of the ventricular chamber. In certain such embodiments, in order to time its pacing of the first cardiac chamber during the second period of time, based on the compensation offset determined and stored during the first period of time, the controller is configured to determine an expected time at which the second LP delivers pacing stimulation to the second cardiac chamber; and at a compensated ventricular-atrial (VA) interval, following the expected time at which the second LP delivers pacing stimulation to the second cardiac chamber, control the pulse generator to deliver pacing stimulation to the first cardiac chamber. The compensated VA interval is based on the compensation offset and a programmed VA interval. In certain such embodiments, the compensated VA interval is equal to the programmed VA interval minus the compensation offset.

In an embodiment, during the first period of time, the LP and the second LP collectively perform pacing in accordance with a DOO mode.

In an embodiment, during the second period of time the receiver does not receive event messages from the second LP for at least one of the following reasons: extrinsic interference prevents the LP from successfully receiving the event messages from the second LP; the second LP abstains from transmitting the event messages to conserve energy; the LP disables at least a portion of the receiver to conserve energy; or a communication channel between the first and second LPs is unstable.

An aspect of the present technology is related to a multi-chamber leadless pacemaker system comprising the LP according to any of the above-described embodiments and the second LP.

Another aspect of the present technology is related to a multi-chamber leadless pacemaker system comprising a first LP and a second LP. The first LP is configured to communicate with the second LP using i2i communication. The first LP is configured to be implanted in or on a first cardiac chamber and to perform pacing of the first cardiac chamber. The second LP is configured to be implanted in or on a second cardiac chamber and to perform pacing of the second cardiac chamber. The second LP comprising a plurality of electrodes, a pulse generator, and a controller. The pulse generator is configured to deliver pacing pulses using at least two of the plurality of electrodes and transmit event messages to the first LP using i2i communication. The controller is communicatively coupled to the pulse generator. The first LP comprises a receiver configured to receive event messages from the second LP using i2i communication. The first LP also comprises a plurality of electrodes, a memory, a pulse generator configured to deliver pacing pulses using at least two of the plurality of electrodes, and a controller communicatively coupled to the receiver, the pulse generator, and the memory. The controller of the first LP is configured to, during a first period of time, during which event messages are received by the receiver from the second LP using i2i communication, control the pulse generator to time pacing of the first cardiac chamber based on the event messages received by the receiver from the second LP using i2i communication so that atrioventricular (AV) synchrony is maintained during the first period of time. The controller of the first LP is also configured to, during the first period of time, during which the event messages are received by the receiver from the second LP using i2i communication, determine and store one or more count values in the memory. The controller of the first LP is also configured to determine a compensation offset based on at least one of the one or more count values determined and stored during the first period of time, wherein the compensation offset is indicative of a drift between timing circuitry of the first LP and timing circuitry of the second LP. Additionally, the controller of the first LP is configured to, during a second period of time, during which event messages are not received by the receiver from the second LP, control the pulse generator to time pacing of the first cardiac chamber based on the compensation offset, to thereby compensate for the drift between the timing circuitry of the first LP and the timing circuitry of the second LP so that the AV synchrony is maintained during the second period of time.

Another aspect of the present technology relates to one of an atrial LP (aLP) and a ventricular LP (vLP) using a compensation offset to compensate for drift between timing circuitry of the aLP and timing circuitry of the vLP, while the aLP and the vLP are providing for AAI+VVI operation, so that synchronization between atrial and ventricular pacing is maintained whenever the vLP delivers ventricular pacing during two or more consecutive cardiac cycles. More specifically, certain embodiments of the present technology are directed to or for use with a multi-chamber LP system that is configured to provide AAI+VVI operation at least some of the time. Such a multi-chamber LP system can be a dual chamber LP system including an aLP configured to be implanted in or on an atrial chamber and a vLP configured to be implanted in or on a ventricular chamber or proximate a left bundle branch (LBB), wherein each of the aLP and the vLP have a respective timing circuitry. In such a system, during a first period of time, the aLP and the vLP are configured to transmit event messages to one another and at least one of the aLP or the vLP is configured to determine and store one or more count values. During a second period of time, the aLP and the vLP are configured to abstain from transmitting event messages to one another to thereby conserve power, the aLP is configured to provide AAI operation, and the vLP is configured to provide VVI operation. During at least one of the first period of time or the second period of time, at least one of the aLP or the vLP is configured to determine a compensation offset based on at least one of the one or more count values determined and stored during the first period of time, wherein the compensation offset is indicative of a drift between the timing circuitry of the aLP and the timing circuitry of the vLP. Additionally, during the second period of time, at least one of the aLP is configured to use the compensation offset while providing the AAI operation, or the vLP is configured to use the compensation offset while providing the VVI operation, to compensate for the drift between the timing circuitry of the aLP and the timing circuitry of the vLP.

In an embodiment, during the second period of time, when the aLP is configured to provide the AAI operation, the aLP is configured to perform atrial pacing when an intrinsic atrial event is not detected within a specified AA interval following a previous paced or intrinsic atrial event, perform atrial sensing, and inhibit the atrial pacing when the intrinsic atrial event is detected within the specified AA interval following the previous paced or intrinsic atrial event. Additionally, during the second period of time, when the vLP is configured to provide the VVI operation, the vLP is configured to perform ventricular pacing when an intrinsic ventricular event is not detected within a specified VV interval following a previous paced or intrinsic ventricular event, perform ventricular sensing, and inhibit the ventricular pacing when the intrinsic ventricular event is detected within the specified VV interval following the previous paced or intrinsic ventricular event.

In an embodiment, during the first period of time, the aLP and the vLP are configured to collectively provide DOO operation.

In an embodiment, the vLP is configured to store the one or more count values during the first period of time, the vLP is configured to determine the compensation offset, and the vLP is configured to use the compensation offset during the second period of time, while the aLP and the vLP are configured to abstain from transmitting event messages to one another to thereby conserve power, the aLP is configured to provide the AAI operation, and the vLP is configured to provide the VVI operation.

In an embodiment, the vLP is configured to use the compensation offset during the second period of time, while that aLP and the vLP are configured to abstain from transmitting event messages to one another to thereby conserve power, the aLP is configured to provide the AAI operation, and the vLP is configured to provide the VVI operation, enables the vLP to maintain synchronization between atrial and ventricular pacing during one or more portions of the second period of time during which the vLP is configured to provide ventricular pacing for two or more consecutive cardiac cycles while providing the VVI operation.

In an embodiment, the vLP is configured to determine and store a measured count value corresponding to a duration between when the vLP delivers pacing stimulation to the ventricular chamber and when the vLP receives an event message, from the aLP, informing the vLP that the aLP delivered or is about to deliver pacing stimulation to the atrial chamber. Additionally, the vLP is configured to determine the compensation offset by subtracting the measured count value from an expected count value, wherein the expected count value corresponds to what the measured count value would be if there was no drift between the timing circuitry of the aLP and the timing circuitry of the vLP.

In an embodiment, the vLP is configured to determine and store each of the following during the first period of time: a beginning low-resolution count value (B_low_res) of a low-resolution counter and a beginning high-resolution count value (B_high_res) of a high-resolution counter corresponding to when the vLP delivers pacing stimulation to ventricular chamber; a measured low-resolution count value (M_low_res) of the low-resolution counter and a measured high-resolution count value (M_high_res) of the high-resolution counter corresponding to when the vLP receives one of the event messages, from the aLP, informing the vLP that the aLP delivered or is about to deliver pacing stimulation to the atrial chamber; a remaining low-resolution count value (R_low_res) corresponding to a base rate low-resolution count value (BR_low_res) minus the measured low-resolution count value (M_low_res); and an expected high-resolution count value (E_high_res) determined based on a sum of the beginning high-resolution count value (B_high_res) and a calculated high-resolution count value (C_high_res). In such an embodiment, the base rate low-resolution count value (BR_low_res) corresponds to how many counts of the low-resolution counter occur during a programmed VV interval, and the calculated high-resolution count value (C_high_res) corresponds to the programmed VV interval minus a programmed AV interval. Additionally, in such an embodiment, the vLP is configured to determine as the compensation offset a result of the expected high-resolution count value (E_high_res) minus the measured high-resolution count value (M_high_res).

In an embodiment, the vLP is configured to: start when the vLP delivered pacing stimulation to the ventricular chamber during an immediately preceding cardiac cycle, serially one after another in any order, using the low-resolution counter to count to a calculated low-resolution count value (C_low_res), using the high-resolution counter to count to the compensation offset, and using the low-resolution counter to count to the remaining low-resolution count value (R_low_res). In such an embodiment, the calculated low-resolution count value (C_low_res) is equal to the measured low-resolution count value (M_low_res) minus the beginning low-resolution count value (B_low_res). Optionally, the vLP is configured to use the low-resolution counter to count to the calculated low-resolution count value (C_low_res) and to count to the remaining low-resolution count value (R_low_res) by using the low-resolution counter to count to a sum of the calculated low-resolution count value (C_low_res) and the remaining low-resolution count value (R_low_res). Such an embodiment also includes the vLP is configured to deliver further pacing stimulation to the ventricular chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order, if the vLP has not detected an intrinsic ventricular event prior to finishing all of the aforementioned counting.

In accordance with an embodiment, during the first period of time, a plurality of the compensation offsets are determined, and during the second period of time an average of the plurality of compensation offsets is used as the compensation offset to compensate for the drift between the timing circuitry of the aLP and the timing circuitry of the vLP.

In accordance with an embodiment, the compensation offset is determined during the first period of time and is used during the second period of time, or the compensation offset both determined and used during the second period of time.

An aspect of the present technology is related to a ventricular leadless pacemaker (vLP) configured to communicate with an atrial LP (aLP) using implant-to-implant (i2i) communication, wherein the vLP is configured to be implanted in or on a ventricular chamber or proximate a left bundle branch (LBB) and to perform pacing of the ventricular chamber, and wherein the aLP is configured to be implanted in or on an atrial chamber and to perform pacing of the atrial chamber. In an embodiment, the vLP comprises: a receiver configured to receive event messages from the aLP using i2i communication; a plurality of electrodes; a memory; a timing circuitry; a pulse generator configured to deliver pacing pulses using at least two of the plurality of electrodes; and a controller communicatively coupled to the receiver, the pulse generator, the timing circuitry, and the memory. In an embodiment, the controller configured to: during a first period of time, during which the aLP and the vLP transmit event messages to one another, determine and store one or more count values in the memory; control the pulse generator to cause the vLP to provide VVI operation during a second period of time during which the aLP and the vLP abstain from transmitting event messages to one another to thereby conserve power, and during which the aLP provides AAI operation; during at least one of the first period of time or the second period of time, determine a compensation offset based on at least one of the one or more count values determined and stored during the first period of time, wherein the compensation offset is indicative of a drift between timing circuitry of the aLP and the timing circuitry of the vLP; and during the second period of time, whenever the vLP is to deliver pacing stimulation to the ventricular chamber to provide the VVI operation and had delivered pacing stimulation to the ventricular chamber during an immediately preceding cardiac cycle, control the pulse generator to time pacing of the ventricular chamber based on the compensation offset, to thereby compensate for the drift between the timing circuitry of the aLP and the timing circuitry of the vLP so that the synchronization between atrial and ventricular pacing is maintained whenever the vLP provides ventricular pacing for two or more consecutive cardiac cycles during the second period of time. The controller is configured to: determine and store, in the memory, a measured count value corresponding to a duration between when the controller controls the pulse generator to deliver pacing stimulation to the ventricular chamber and when the receiver receives an event message, from the aLP, informing the vLP that the aLP delivered or is about to deliver pacing stimulation to the atrial chamber; and determine the compensation offset by subtracting the measured count value from an expected count value, wherein the expected count value corresponds to what the measured count value would be if there was no drift between the timing circuitry of the aLP and the timing circuitry of the vLP.

In an embodiment, the vLP further comprises: a low-resolution counter; and a high-resolution counter. During the first period of time, the controller is configured to determine and store, in the memory, each of the following: a beginning low-resolution count value (B_low_res) of a low-resolution counter and a beginning high-resolution count value (B_high_res) of a high-resolution counter corresponding to when the controller controls the pulse generator to deliver pacing stimulation to the ventricular chamber; a measured low-resolution count value (M_low_res) of the low-resolution counter and a measured high-resolution count value (M_high_res) of the high-resolution counter corresponding to when the receiver receives one of the event messages, from the aLP, informing vLP that the aLP delivered or is about to deliver pacing stimulation to the atrial chamber; a remaining low-resolution count value (R_low_res) corresponding to a base rate low-resolution count value (BR_low_res) minus the measured low-resolution count value (M_low_res); and an expected high-resolution count value (E_high_res) determined based on a sum of the beginning high-resolution count value (B_high_res) and a calculated high-resolution count value (C_high_res). In such an embodiment, the base rate low-resolution count value (BR_low_res) corresponds to how many counts of the low-resolution counter occur during a programmed VV interval. Additionally, the calculated high-resolution count value (C_high_res) corresponds to the programmed VV interval minus a programmed AV interval. The controller is also configured to determine as the compensation offset the expected high-resolution count value (E_high_res) minus the measured high-resolution count value (M_high_res).

In an embodiment, the high-resolution counter comprises a modulo-n counter that is configured to count from zero to n-1 and then reset back to zero, and the controller is configured to determine that the expected high-resolution count value (E_high_res) is equal to a modulo-n operation on the sum of the beginning high-resolution count value (B_high_res) and the calculated high-resolution count value (C_high_res).

In an embodiment, in order to time pacing of the ventricular chamber whenever the vLP delivered pacing stimulation to the ventricular chamber during an immediately preceding cardiac cycle during the second period of time, based on the compensation offset that the LP determined and stored, the controller is configured to: starting when the controller most recently controlled the pulse generator to deliver pacing stimulation to the ventricular chamber during the immediately preceding cardiac cycle, use the low-resolution counter to count to a calculated low-resolution count value (C_low_res) that is equal to the measured low-resolution count value (M_low_res) minus the beginning low-resolution count value; starting when the low-resolution counter finishes counting to the calculated low-resolution count value (C_low_res), use the high-resolution counter to count to the compensation offset; starting when the high-resolution counter finishes counting to the compensation offset, use the low-resolution counter to count to the remaining low-resolution count value (R_low_res); and when the low-resolution counter finishes counting to the remaining low-resolution count value (R_low_res), control the pulse generator to deliver further pacing stimulation to the ventricular chamber.

More generally, in an embodiment, in order to time pacing of the ventricular chamber whenever the vLP delivered pacing stimulation to the ventricular chamber during an immediately preceding cardiac cycle during the second period of time, based on the compensation offset that the LP determined and stored, the controller is configured to: starting when the controller most recently controlled the pulse generator to deliver pacing stimulation to the ventricular chamber, serially one after another in any order, use the low-resolution counter to count to a calculated low-resolution count value (C_low_res), use the high-resolution counter to count to the compensation offset, and use the low-resolution counter to count to the remaining low-resolution count value (R_low_res). In such an embodiment, the calculated low-resolution count value (C_low_res) is equal to the measured low-resolution count value (M_low_res) minus the beginning low-resolution count value (B_low_res). Use of the low-resolution counter to count to the measured low-resolution count value (M_low_res) and to count to the remaining low-resolution count value (R_low_res) may be performed by using the low-resolution counter to count to a sum of the measured low-resolution count value (M_low_res) and the remaining low-resolution count value (R_low_res). The controller is also configured to control the pulse generator to deliver further pacing stimulation to the ventricular chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order.

An aspect of the present technology is related to a multi-chamber leadless pacemaker system comprising an aLP and an vLP configured to communicate with one another using i2i communication, wherein the aLP is configured to be implanted in or on an atrial chamber and to perform pacing of the atrial chamber, and the vLP is configured to be implanted in or on a ventricular chamber or proximate a LBB and to perform pacing of the ventricular chamber. The vLP comprises: a receiver configured to receive event messages from the aLP using i2i communication; a plurality of electrodes; a memory; a timing circuitry; a pulse generator configured to deliver pacing pulses using at least two of the plurality of electrodes; and a controller communicatively coupled to the receiver, the pulse generator, the timing circuitry, and the memory. The controller is configured to: during a first period of time, during which the aLP and the vLP transmit event messages to one another, determine and store one or more count values in the memory. Additionally, the controller is configured to control the pulse generator to cause the vLP to provide VVI operation during a second period of time during which the aLP and the vLP abstain from transmitting event messages to one another to thereby conserve power, and during which the aLP provides AAI operation. Additionally the controller is configured to determine, during at least one of the first period of time or the second period of time, a compensation offset based on at least one of the one or more count values determined and stored during the first period of time, wherein the compensation offset is indicative of a drift between timing circuitry of the aLP and the timing circuitry of the vLP. The controller is also configured to during the second period of time, whenever the vLP is to deliver pacing stimulation to the ventricular chamber to provide the VVI operation and had delivered pacing stimulation to the ventricular chamber during an immediately preceding cardiac cycle, control the pulse generator to time pacing of the ventricular chamber based on the compensation offset, to thereby compensate for the drift between the timing circuitry of the aLP and the timing circuitry of the vLP so that the synchronization between atrial and ventricular pacing is maintained whenever the vLP provides ventricular pacing for two or more consecutive cardiac cycles during the second period of time.

In an embodiment, the controller of the vLP is configured to determine and store, in the memory, a measured count value corresponding to a duration between when the controller controls the pulse generator to deliver pacing stimulation to the ventricular chamber and when the receiver receives an event message, from the aLP, informing the vLP that the aLP delivered or is about to deliver pacing stimulation to the atrial chamber. The controller is also configured to determine the compensation offset by subtracting the measured count value from an expected count value, wherein the expected count value corresponds to what the measured count value would be if there was no drift between the timing circuitry of the aLP and the timing circuitry of the vLP.

In an embodiment, the vLP further comprises a low-resolution counter and a high-resolution counter. During the first period of time, the controller of the vLP is configured to determine and store, in the memory, each of the following: a beginning low-resolution count value (B_low_res) of a low-resolution counter and a beginning high-resolution count value (B_high_res) of a high-resolution counter corresponding to when the controller controls the pulse generator to deliver pacing stimulation to the ventricular chamber; a measured low-resolution count value (M_low_res) of the low-resolution counter and a measured high-resolution count value (M_high_res) of the high-resolution counter corresponding to when the receiver receives one of the event messages, from the aLP, informing vLP that the aLP delivered or is about to deliver pacing stimulation to the atrial chamber; a remaining low-resolution count value (R_low_res) corresponding to a base rate low-resolution count value (BR_low_res) minus the measured low-resolution count value (M_low_res); and an expected high-resolution count value (E_high_res) determined based on a sum of the beginning high-resolution count value (B_high_res) and a calculated high-resolution count value (C_high_res). In such an embodiment, the base rate low-resolution count value (BR_low_res) corresponds to how many counts of the low-resolution counter occur during a programmed VV interval, and the calculated high-resolution count value (C_high_res) corresponds to the programmed VV interval minus a programmed AV interval. The controller is also configured to determine as the compensation offset the expected high-resolution count value (E_high_res) minus the measured high-resolution count value (M_high_res).

In an embodiment, in order to time pacing of the ventricular chamber whenever the vLP delivered pacing stimulation to the ventricular chamber during an immediately preceding cardiac cycle during the second period of time, based on the compensation offset that the LP determined and stored, the controller of the vLP is configured to: starting when the controller most recently controlled the pulse generator to deliver pacing stimulation to the ventricular chamber, serially one after another in any order, use the low-resolution counter to count to a calculated low-resolution count value (C_low_res), use the high-resolution counter to count to the compensation offset, and use the low-resolution counter to count to the remaining low-resolution count value (R_low_res). In such an embodiment, the calculated low-resolution count value (C_low_res) is equal to the measured low-resolution count value (M_low_res) minus the beginning low-resolution count value (B_low_res). In such an embodiment, use of the low-resolution counter to count to the measured low-resolution count value (M_low_res) and to count to the remaining low-resolution count value (R_low_res) may be performed by using the low-resolution counter to count to a sum of the measured low-resolution count value (M_low_res) and the remaining low-resolution count value (R_low_res). The controller is also configured to control the pulse generator to deliver further pacing stimulation to the ventricular chamber, when the low-resolution counter has finished counting to the calculated low-resolution count value (C_low_res) and to the remaining low-resolution count value (R_low_res), or the sum thereof, and the high-resolution counter has finished counting to the compensation offset, serially one after another in any order, if the vLP has not detected an intrinsic ventricular event prior to finishing all of the aforementioned counting.

This summary is not intended to be a complete description of the embodiments of the present technology. Other features and advantages of the embodiments of the present technology will appear from the following description in which the preferred embodiments have been set forth in detail, in conjunction with the accompanying drawings and claims.

### BRIEF DESCRIPTION OF THE DRAWINGS

Embodiments of the present technology relating to both structure and method of operation may best be understood by referring to the following description and accompanying drawings, in which similar reference characters denote similar elements throughout the several views:
FIG. 1 illustrates a system formed in accordance with certain embodiments herein as implanted in a heart.
FIG. 2 is a block diagram of a single LP in accordance with certain embodiments herein.
FIG. 3 illustrates an LP in accordance with certain embodiments herein.
FIG. 4 is a timing diagram demonstrating one embodiment of i2i communication for a paced event.
FIG. 5 is a timing diagram demonstrating one embodiment of i2i communication for a sensed event.
FIG. 6 is a block diagram showing example details of the clock(s) and counter(s) introduced in FIG. 2, in accordance with an embodiment of the present technology.
FIG. 7 is a timing diagram used to illustrate example operations of the aLP and the vLP, introduced in FIG. 1, and the i2i communication therebetween, wherein during a portion of the timing diagram there is low environmental noise that does not adversely affect the i2i communication, and during a further portion of the timing diagram there is high environmental noise which adversely affects the i2i communication.
FIG. 8 is a high level flow diagram used to summarize methods that compensate for the drift between timing circuitry of a first LP and timing circuitry of a second LP so that the AV synchrony is maintained during a period of time during which the first LP does not receive i2i messages from the second LP, wherein the first LP is configured to be implanted in or on a first cardiac chamber and to perform pacing of the first cardiac chamber, and wherein the second LP is configured to be implanted in or on a second cardiac chamber and to perform pacing of the second cardiac chamber.
FIG. 9 is a flow diagram used to describe additional details of one of the steps introduced in FIG. 8. More specifically, FIG. 9 is used to describe how a first LP can determine and store a compensation offset based on values stored during a first period of time during which the first LP receives event messages from the second LP using i2i communication, so that the compensation offset can be used to compensate for drift and maintain AV synchrony during a second period of time during which the first LP does not receive i2i messages from the first LP.
FIG. 10 is a flow diagram used to describe additional details of another one of the steps introduced in FIG. 8. More specifically, FIG. 10 is used to describe how a first LP times its pacing of a first cardiac chamber (in or one which the first LP is implanted) during a second period of time (during which the first LP does not receive i2i communication from the second LP), based on a compensation offset that the first LP determines based on values determined and stored during a first period of time (during which the first LP received event messages from the second LP using i2i communication).
FIG. 11A includes a timing diagram used to illustrate how a vLP can determine a compensation offset, in accordance with an embodiment of the present technology.
FIG. 11B includes a timing diagram used to illustrate how an aLP can determine a compensation offset, in accordance with an embodiment of the present technology.
FIG. 12A is a timing diagram illustrating operations of an aLP and a vLP during a period of time during which there is no i2i communications therebetween, and during which the aLP uses an embodiment of the present technology to compensate for drift between respective timing circuitry of the aLP and the vLP.
FIG. 12B is a timing diagram illustrating operations of an aLP and a vLP during a period of time during which there is no i2i communications therebetween, and during which the vLP uses an embodiment of the present technology to compensate for drift between respective timing circuitry of the vLP and the aLP.
FIG. 13 includes a graph that compares the drift that occurs between an aLP and a vLP without and without using an embodiment of the present technology to compensate for the drift.

### DETAILED DESCRIPTION

Certain embodiments described herein generally relate to devices and systems for providing drift compensation in a multi-chamber LP system that includes two or more leadless pacemakers. A dual chamber LP system is an example of a multi-chamber LP system that includes two LPs, e.g., an atrial LP (aLP) implanted in or on a right atrial chamber (aka the right atrium) and a ventricular LP (vLP) implanted in or on a right ventricular chamber (aka the right ventricle). In certain embodiments, the vLP is implanted proximate a left bundle branch (LBB), in which case a majority of the vLP is implanted within the right ventricular chamber and a distal electrode of the vLP may be inserted into the septum to access the LBB. For the purpose of this discussion, it is assumed that the vLP that is implanted in or on a ventricular chamber or proximate the LBB performs pacing of the ventricular chamber. Accordingly, for the purpose of this discussion, ventricular pacing may be performed by delivering pacing stimulation to the LBB. An example operational mode that the aLP and the vLP can collectively provide is the DOO mode, which provides for dual chamber pacing, i.e., both atrial pacing (that is performed by the aLP) and ventricular pacing (that is performed by the vLP).

In such a dual chamber LP system, the vLP may time its delivery of ventricular pacing stimulation (to the ventricular chamber in or on which the vLP is implanted) such that the ventricular pacing stimulation is delivered at a specified atrioventricular interval (AVI) after an atrial pacing stimulation was delivered by the aLP. Similarly, the aLP may time its delivery of atrial pacing stimulation (to the atrial chamber in or one which the aLP is implanted) such that the atrial pacing stimulation is delivered at a specified ventricular-atrial interval (VAI) is delivered after a ventricular pacing stimulation was delivered by the vLP. Such operation of the dual chamber LP system may occur, e.g., when the dual chamber LP system is in a DOO mode. However, where electromagnetic interference (EMI) and/or other noise is present, which prevents aLP and the vLP from successfully performing i2i communication, the dual chamber LP system may revert to a VOO mode, during which only the vLP delivers pacing stimulation. In other words, EMI and/or other noise may cause a dual chamber LP system that is operating in a dual chamber pacing mode, such as the DOO mode, to transition to a signal chamber pacing mode, such as the VOO mode.

In a dual-chamber LP system (or other type of multi-chamber LP system), each LP includes its own respective timing circuitry, which can include one or more clock signal generators, one or more counters, and/or the like, which enables the LP to time its various operations. For example, when a dual chamber LP system is operating in the DOO mode, the vLP uses its timing circuitry to deliver ventricular pacing stimulation at a specified AVI after an atrial pacing stimulation was delivered by the aLP, and the aLP uses its timing circuitry to deliver atrial pacing stimulation at a specified VAI after a ventricular pacing stimulation was delivered by the vLP. As just noted above, EMI and/or other noise may cause a dual chamber LP system that is operating in the DOO mode, to transition to the VOO mode. This is in part because the EMI and/or other noise prevents the aLP and the vLP from successfully performing i2i communication, and the aLP and the vLP rely on successful i2i communication to maintain appropriate AV synchrony.

When the aLP and the vLP are unable to successfully perform i2i communication, e.g., due to EMI and/or other noise, rather than transitioning from the DOO mode to the VOO mode, the aLP and the vLP can attempt to rely on their own respectively timing circuitry to continue to collectively operate in the DOO mode, during which dual chamber pacing is performed. More specifically, the aLP can time its delivery of atrial pacing stimulation using its own timing circuitry such that the atrial chamber (in or on which the aLP is implanted) is paced in accordance with an AA interval corresponding to a base pacing rate, and the vLP can time its deliver of ventricular pacing stimulation using its own timing circuitry such that the ventricular chamber (in or on which the vLP is implanted) is paced in accordance with a VV interval corresponding to the base pacing rate. While this type of dual chamber pacing without i2i communication may work in theory, that may not be the case in the real world due to drift between the timing circuitry of the aLP and the timing circuitry of the vLP. More specifically, because the aLP and the vLP include their own respective timing circuitry, which drift relative to one another over time, if the aLP and the vLP respectively deliver atrial pacing stimulation and ventricular pacing stimulation independent of one another, the dual chamber LP system may not reliably maintain AV synchrony. This may result in the vLP delivering pacing stimulation to the ventricular chamber (in or on which the vLP is implanted) at a time that is significantly earlier than (or significantly later than) a specified AVI following when the aLP delivered atrial stimulation to the atrial chamber (in or on which the aLP is implanted), which is undesirable and may lead to poor patient outcomes. Similarly, this may result in the aLP delivering pacing stimulation to the atrial chamber (in or on which the aLP is implanted) at a time that is significantly earlier than (or significantly later than) a specified VAI following when the vLP delivered ventricular stimulation to the ventricular chamber (in or on which the vLP is implanted), which is also undesirable and may lead to poor patient outcomes.

Certain embodiments of the present technology compensate for the drift between the timing circuitry of the vLP and the timing circuitry of the aLP so that AV synchrony is maintained during a period of time, during which the vLP does not receive i2i messages from the aLP. More generally, certain embodiments of the present technology compensate for the drift between timing circuitry of a first LP and timing circuitry of a second LP so that the AV synchrony is maintained during a period of time during which the first LP does not receive i2i messages from the second LP. As will be described in additional detail below, in accordance with certain embodiments, the aforementioned drift is compensated for by at least one of the LPs (and possibly both of the LPs) determining and storing a compensation offset based on one or more count values determined during a period of time that the first LP receives event messages from the second LP, and using the compensation offset to compensate for the drift during a further period of time that the first LP does not receive event messages from the second LP.

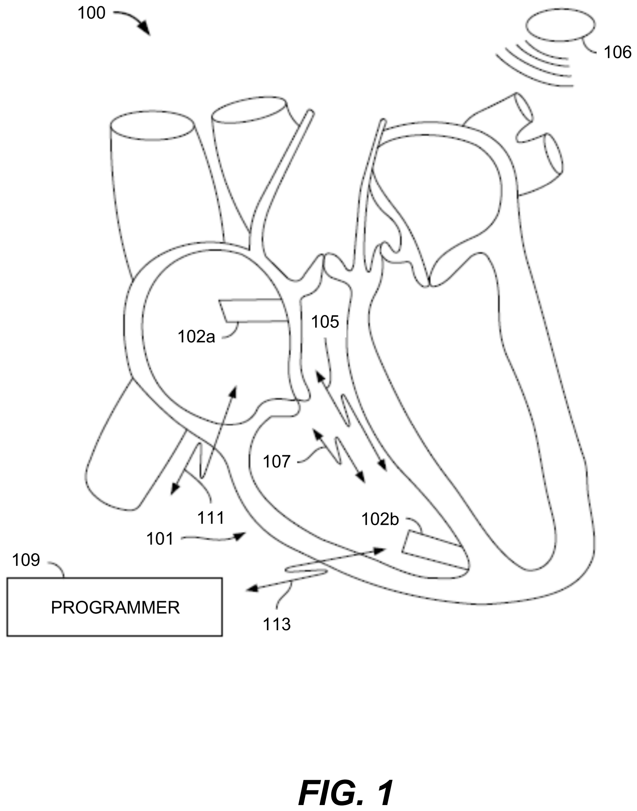

Before providing additional details of the specific embodiments of the present technology mentioned above, an example system in which embodiments of the present technology can be used will first be described with reference to FIGS. 1-5. More specifically, FIGS. 1-5 are used to describe an exemplary multi-chamber leadless pacemaker system with which embodiments of the present technology can be used. A leadless pacemaker can also be referred to herein as a leadless cardiac pacemaker, or more succinctly as an LP. Where a cardiac pacing system includes a non-vascular implantable cardioverter-defibrillator (NV-ICD), such as a subcutaneous-ICD (S-ICD), the NV-ICD may perform certain sensing operations and may communicate with one or more LPs by sending and/or receiving messages to and/or from one or more LPs, as can be appreciated from the below discussion. Where a cardiac pacing system includes a programmer, the programmer may be used to program one or more IMDs (e.g., LPs), download information to one or more IMDs, and/or upload information from one or more IMDs, as can be appreciated from the below description.

FIG. 1 illustrates a system 100 that includes LPs 102a and 102b located in different chambers of a heart 101. The portion of the system 100 that includes the LPs 102a and 102b can be referred to as a multi-chamber LP system 100. The LP 102a is located in a right atrium, and thus, is an example of an atrial LP (aLP). The LP 102b is located in a right ventricle, and thus, is an example of a ventricular LP (vLP). In certain embodiments, the vLP 102b is implanted proximate a left bundle branch (LBB) and delivers stimulation pulses to the LBB which causes ventricular pacing, and thus, such pacing pulses can be considered ventricular pacing pulses. The LPs 102a and 102b can communicate with one another to inform one another of various local physiologic activities, such as local intrinsic events, local paced events, and/or the like. The LPs 102a and 102b may be constructed in a similar manner, but operate differently based upon which chamber the LP 102a or 102b is located. The LPs 102a and 102b may sometimes be referred to collectively herein as the LPs 102, or individually as an LP 102.

In certain embodiments, the LPs 102a and 102b communicate with one another, see 105 and 107, and/or with an NV-ICD 106, such as by conductive communication through the same electrodes that are used for sensing and/or delivery of pacing therapy. The LPs 102a and 102b may also be able to use conductive communication 111 and 113 to communicate with an external device, e.g., a programmer 109, having electrodes placed on the skin of a patient within with the LPs 102a and 102b are implanted. It is noted that the term "conductive communication" and the term "conducted communication" are used interchangeably herein. While not shown in FIG. 1 (and not preferred, since it would increase the size and power consumption of the LPs 102a and 102b), the LPs 102a and 102b can potentially include an antenna and/or telemetry coil that would enable them to communicate with one another, the NV-ICD 106 and/or an external device, such as the programmer 109, using RF and/or inductive communication. While only two LPs 102 are shown in FIG. 1, it is possible that more than two LPs 102 can be implanted in a patient. For example, to provide for bi-ventricular pacing and/or cardiac resynchronization therapy (CRT), in addition to having LPs 102a and 102b implanted in the right atrial (RA) chamber and the right ventricular (RV) chamber, a further LP can be implanted in the left ventricular (LV) chamber. Additionally, or alternatively, a further LP can be implanted in the left atrial (LA) chamber.

In some embodiments, the LPs 102 can be co-implanted with the ICD 106. Each LP 102 uses two or more electrodes located within, on, or within a few centimeters of the housing of the LP 102, for pacing and sensing at the cardiac chamber, and additionally for bidirectional communication 105, 107, 111, 113 with one another, with the programmer 109, and the ICD 106.

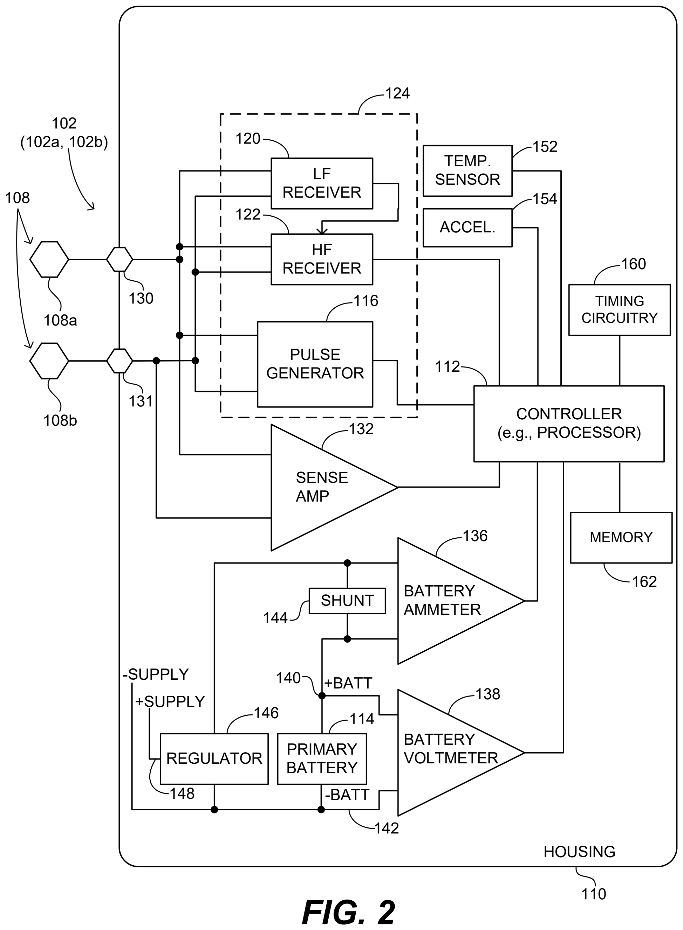

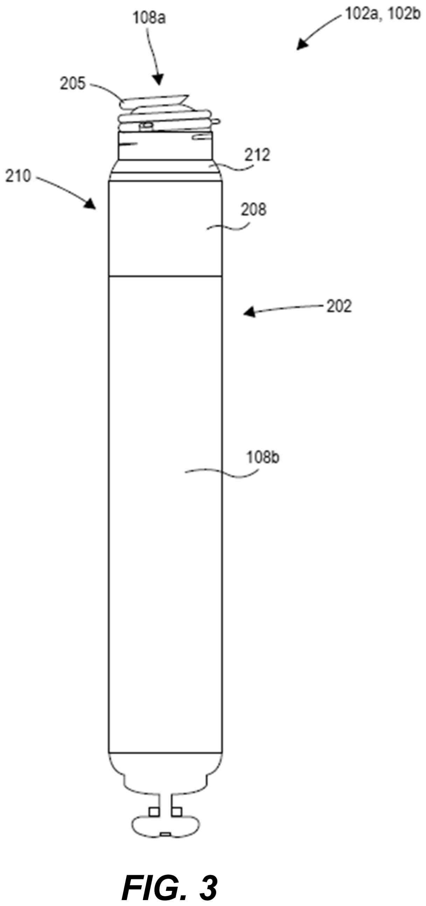

Referring to FIG. 2, a block diagram shows an embodiment for portions of the electronics within LPs 102a, 102b configured to provide conductive communication through the sensing/pacing electrodes 108a, 108b. One or more of LPs 102a and 102b include at least two leadless electrodes 108a, 108b configured for delivering cardiac pacing pulses, sensing evoked and/or natural cardiac electrical signals, and unidirectional or bi-directional communication. In FIG. 2 (and FIG. 3) the two electrodes shown therein are labeled 108a and 108b. Such electrodes can be referred to collectively as the electrodes 108, or individually as an electrode 108. An LP 102, or other type of IMD, can include more than two electrodes 108, depending upon implementation.

In FIG. 2, each of the LPs 102a, 102b is shown as including first and second receivers 120 and 122 that collectively define separate first and second communication channels 105 and 107 (FIG. 1), (among other things) between LPs 102a and 102b. Although first and second receivers 120 and 122 are depicted, in other embodiments, each LP 102a, 102b may include only one of the receivers 120, 122, or may include additional receivers other than first and second receivers 120 and 122. As will be described in additional detail below, the pulse generator 116 can function as a transmitter that transmits i2i communication signals using the electrodes 108. In certain embodiments, LPs 102a and 102b may communicate over more than just first and second communication channels 105 and 107. In certain embodiments, LPs 102a and 102b may communicate over one common communication channel 105. More specifically, LPs 102a and 102b can communicate conductively over a common physical channel via the same electrodes 108 that are also used to deliver pacing pulses. Usage of the electrodes 108 for communication enables the one or more LPs 102a and 102b to perform antenna-less and telemetry coil-less communication.

The receivers 120 and 122 can also be referred to, respectively, as a low frequency (LF) receiver 120 and a high frequency (HF) receiver 122, because the receiver 120 is configured to monitor for one or more signals within a relatively low frequency range (e.g., below 250 kHz) and the receiver 122 is configured to monitor for one or more signals within a relatively high frequency range (e.g., above 250 kHz). In certain embodiments, the receiver 120 (and more specifically, at least a portion thereof) is always enabled and monitoring for a wakeup notice, which can simply be a wakeup pulse, within a specific low frequency range (e.g., between 1 kHz and 250 kHz); and the receiver 122 is selectively enabled by the receiver 120. The receiver 120 is configured to consume less power than the receiver 122 when both the first and second receivers are enabled. Accordingly, the receiver 120 can also be referred to as a low-power receiver 120, and the receiver 122 can also be referred to as a high-power receiver 122. The low-power receiver 120 is incapable of receiving signals within the relatively high frequency range (e.g., above 250 kHz), but consumes significantly less power than the high-power receiver 122. This way the low-power receiver 120 is capable of always monitoring for a wakeup notice without significantly depleting the battery (e.g., 114) of the LP 102. In accordance with certain embodiments, the high-power receiver 122 is selectively enabled by the low-power receiver 120, in response to the low-power receiver 120 receiving a wakeup notice, so that the high-power receiver 122 can receive the higher frequency signals, and thereby handle higher data throughput needed for effective i2i communication without unnecessarily and rapidly depleting the battery 114 of the LP 102 (which the high-power receiver 122 may do if it were always enabled).

In accordance with certain embodiments, when one of the LPs 102a and 102b senses an intrinsic event or delivers (or is about to deliver) a paced event, the corresponding LP 102a, 102b can transmit an event message to the other LP 102a, 102b. Where an event message originates from an LP (e.g., 102a) implanted in or on an atrial chamber (e.g., the right atrial chamber), the event message can be referred to more specifically as an atrial event message. Where an event message originates from an LP (e.g., LP 102b) implanted in or on a ventricular chamber (e.g., the right ventricular chamber) or proximate the LBB, the event message can be referred to more specifically as a ventricular event message.

For example, when an atrial LP 102a senses or paces an atrial event, the atrial LP 102a transmits an atrial event message including an event marker indicative of a nature of the event (e.g., intrinsic/sensed atrial event, paced atrial event). When a ventricular LP 102b senses or paces a ventricular event, the ventricular LP 102b transmits a ventricular event message including an event marker indicative of a nature of the event (e.g., intrinsic/sensed ventricular event, paced ventricular event). In certain embodiments, each LP 102a, 102b can transmit a paced event message to the other LP 102a, 102b preceding delivery of an actual pace pulse so that the remote LP can blank its sense inputs in anticipation of that remote pace pulse (to prevent inappropriate crosstalk sensing by the remote LP). In alternative embodiments, each LP 102a, 102b may abstain from transmitting a paced event message to the other LP 102a, 102b following delivery of a pace pulse, and the remote LP relies on an alternative technique for avoiding crosstalk sensing.

Where an event message is transmitted by a first LP to a second LP to inform the second LP of an intrinsic event sensed by the first LP, the event message can be referred to more specifically as a sensed event message. Where the first LP is an aLP (e.g., 102a), the sensed event message can be referred to more specifically as an atrial sensed event message. Where the first LP is a vLP (e.g., 102a), the sensed event message can be referred to more specifically as a ventricular sensed event message. Where an event message is transmitted by a first LP to a second LP to inform the second LP of a paced event caused (or about to be caused) by the first LP, the event message can be referred to more specifically as a paced event message. Where the first LP is an aLP (e.g., 102a), the paced event message can be referred to more specifically as an atrial paced event message. Where the first LP is a vLP (e.g., 102a), the paced event message can be referred to more specifically as a ventricular paced event message.

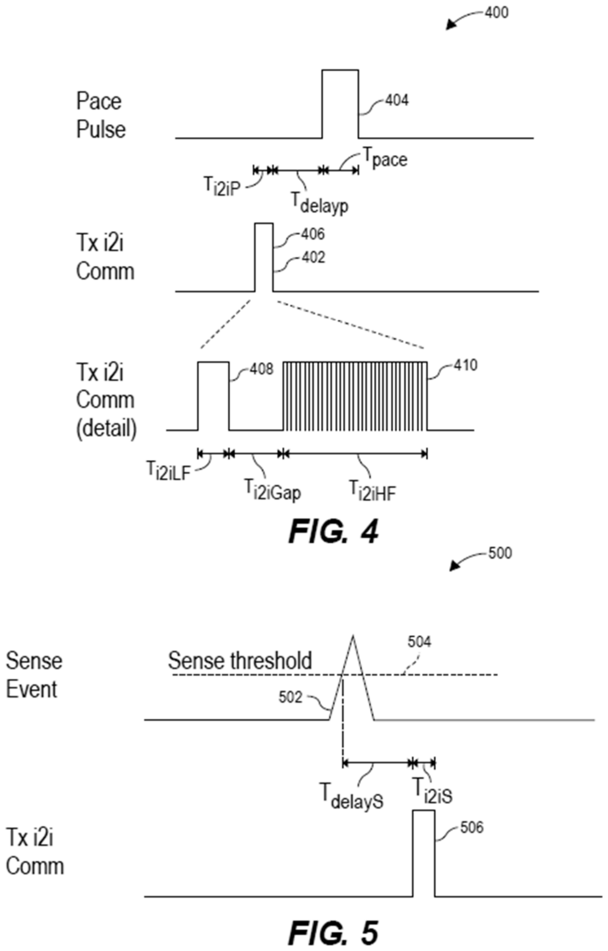

The implant event messages, which are also referred to herein as cardiac event messages or event messages, may be formatted in various manners. As one example, each event message may include a leading trigger pulse 408, see Fig. 4, (also referred to as an LP wakeup notice, notice trigger pulse, wakeup pulse or wakeup signal) followed by an event marker, such as in the form of a pulse train 410 as shown in Fig. 4. The trigger pulse 408 is transmitted over a first channel 105 (e.g., with a pulse duration of approximately 4 µs to approximately 1.0 msec and/or within a fundamental frequency range of approximately 1 kHz to approximately 250 kHz). The trigger pulse 408 indicates that an event marker is about to be transmitted over a second channel 107 (e.g., within a higher frequency range). The event marker can then be transmitted over the second channel 107.

The event markers may include data indicative of one or more events (e.g., a sensed intrinsic atrial activation for an atrial located LP, a sensed intrinsic ventricular activation for a ventricular located LP). The event markers may include different markers for intrinsic and paced events. The event markers may also indicate start or end times for timers (e.g., an AV interval, a blanking interval, etc.). Optionally, the implant event message may include a message segment that includes additional/secondary information.

Optionally, the LP (or other IMD) that receives any i2i communication signal from another LP (or other IMD) or from an external device, such as a programmer, may transmit a receive acknowledgement indicating that the receiving LP (or other IMD) received the i2i communication signal. In certain embodiments, where an LP (or other IMD) expects to receive an i2i communication signal within a window, and fails to receive the i2i communication signal within the window, the LP (or other IMD) may transmit a failure-to-receive acknowledgement indicating that the receiving LP (or other IMD) failed to receive the i2i communication signal. Other variations are also possible and within the scope of the embodiments described herein.

The event messages enable the LPs 102a, 102b to deliver synchronized therapy and additional supportive features (e.g., measurements, etc.). To maintain coordinated therapy, each of the LPs 102a and 102b can be made aware (through the event messages) when an event occurs in the chamber containing the other LP 102a, 102b. Some embodiments described herein provide efficient and reliable processes to maintain synchronization between LPs 102a and 102b without maintaining continuous communication between LPs 102a and 102b. In accordance with certain embodiments herein, low-power event messages/signaling may be maintained between LPs 102a and 102b synchronously or asynchronously.

For synchronous event signaling, LPs 102a and 102b may maintain synchronization and regularly communicate at a specific interval. Synchronous event signaling allows the transmitter (e.g., pulse generator 116) and receivers 120, 122 in each LP 102a, 102b to use limited (or minimal) power as each LP 102a, 102b is only powered for a small fraction of the time in connection with transmission and reception. For example, LP 102a, 102b may transmit/receive (Tx/Rx) communication messages in time slots having duration of 10-20 µs, where the Tx/Rx time slots occur periodically (e.g., every 10-20 ms).

In accordance with certain embodiments herein, LPs 102a and 102b may utilize multi-stage receivers 120, 122 that implement a staged receiver wakeup scheme in order to improve reliability yet remain power efficient. Each of LPs 102a and 102b may include first and second receivers 120 and 122 that operate with different first and second activation protocols and different first and second receive channels. For example, first receiver 120 may be assigned a first activation protocol that is "always on" (also referred to as always awake) and that listens over a first receive channel that has a lower fundamental frequency range/pulse duration as compared to the fundamental frequency range assigned to the second receive channel.

In accordance with certain embodiments, the first receiver 120 may maintain the first channel active (awake) at all times (including when the second channel is inactive (asleep)) in order to listen for messages from a remote LP. The second receiver 122 may be assigned a second activation protocol that is a triggered protocol, in which the second receiver 122 becomes active (awake) in response to detection of trigger events over the first receive channel (e.g., when the incoming signal corresponds to the LP wakeup notice, activating the second channel at the local LP). The terms active, awake and enabled are used interchangeably herein.

Still referring to FIG. 2, each LP 102a, 102b is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. In FIG. 2, each LP 102a, 102b is shown as having memory 162 that is communicatively coupled to the controller 112. Such memory 162 can be external to the controller 112 or can be part of the controller 112. As will be described in additional detail below, the memory 162 can be used to store various count and compensation offset values. It would also be possible for such values, or at least some of these values, to be stored in one or more registers rather than in memory 162.

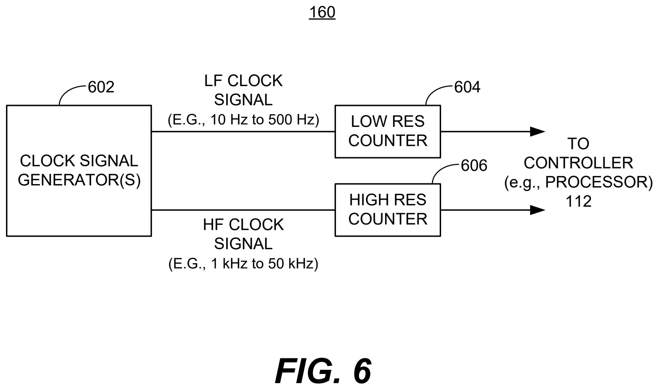

In FIG. 2, each LP 102a, 102b is shown as including timing circuitry 160, which is shown as being communicatively coupled to the controller 112 to thereby provide one or more clock signals and one or more counter signals to the controller 112. For example, each LP 102a, 102b can include a crystal oscillator. Alternatively, or additionally, each LP 102a, 102b can include a non-crystal oscillator, which can be an RC oscillator, such as a phase shift oscillator or Wien bridge oscillator, an LC oscillator, such as a Colpitts oscillator, a Hartley oscillator or a Clapp oscillator, a voltage controlled oscillator (VCO), such as harmonic oscillator or a relaxation oscillator, or a ring oscillator, such as a basic ring oscillator, a differential ring oscillator, a current-starved ring oscillator or voltage-controller ring oscillator, but is not limited thereto. More generally, each LP 102a, 102b can include respective timing circuitry 160 that produces one or more timing signals, which can include clock signals and counter signals generated by counters that count rising or falling edges of the clock signals, as will be described in additional detail below, e.g., with reference to FIG. 6. It is also possible that the clock(s) and counter(s) can be implemented by the controller 112 itself. In other words, the timing circuitry 160, or at least a portion thereof, can be implemented within the controller 112.

The controller 112 can further include, e.g., timing control circuitry to control the timing of the stimulation pulses (e.g., pacing rate, atrioventricular (AV) interval, atrial interconduction (AA) interval, or ventricular interconduction (VV) interval, etc.). For example, the controller 112 of the vLP 102b can be used to implement one or more timers, including but not limited to, an AV interval timer and a ventricular-to-ventricular interval (VV interval) timer. The controller 112 of the aLP 102a can similarly be used to implement one or more timers that may be used by the aLP 102a. Such timing control circuitry may also be used for the timing of refractory periods, blanking intervals, noise detection windows, evoked response windows, alert intervals, marker channel timing, and so on. Where a timer is used to count up (or count down), the timer may also be referred to herein as a timer counter, or more succinctly, as a counter.

The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LP 102a, 102b is located, such as when the associated chamber is not in a refractory state. In addition, a LP 102a, 102b that receives an event message may enter an "event refractory" state (or event blanking state) following receipt of the event message. The event refractory/blanking state may be set to extend for a determined period of time after receipt of an event message in order to avoid the receiving LP 102a, 102b from inadvertently sensing another signal as an event message that might otherwise cause retriggering. For example, the receiving LP 102a, 102b may detect a measurement pulse from another LP 102a, 102b or programmer 109.

In accordance with certain embodiments herein, programmer 109 may communicate over a programmer-to-LP channel, with LP 102a, 102b utilizing the same communication scheme. The external programmer may listen to the event message transmitted between LP 102a, 102b and synchronize programmer to implant communication such that programmer 109 does not transmit communication signals 113 until after an implant to implant messaging sequence is completed.

While not shown, a communication capacitor can be provided in LP 102a, 102b. The communication capacitor may be used to transmit event signals having higher voltage for the event message pulses to improve communication, such as when the LPs 102a and 102b experience difficulty sensing event messages. The high voltage event signaling may be used for implants with high signal attenuation or in the case of a retry for an ARQ (automatic repeat request) handshaking scheme.

In some embodiments, the individual LP 102a can comprise a hermetic housing 110 configured for placement on or attachment to the inside or outside of a cardiac chamber and at least two leadless electrodes 108 proximal to the housing 110 and configured for bidirectional communication with at least one other device 106 within or outside the body.

FIG. 2 depicts a single LP 102a (or 102b) and shows the LP's functional elements substantially enclosed in a hermetic housing 110. The LP 102a (or 102b) has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can further contain circuits for monitoring device health, for example an optional battery current monitor 136 and an optional battery voltage monitor 138, and can contain circuits for controlling operations in a predetermined manner.

The electrodes 108 can be configured to communicate bidirectionally among the multiple LPs 102 and/or the implanted ICD 106 to coordinate pacing pulse delivery and optionally other therapeutic or diagnostic features using messages that identify an event at an individual pacemaker originating the message and a pacemaker receiving the message react as directed by the message depending on the origin of the message. An LP 102a, 102b that receives the event message reacts as directed by the event message depending on the message origin or location. In some embodiments or conditions, the two or more leadless electrodes 108 can be configured to communicate bidirectionally among the one or more LPs 102a and/or the ICD 106 and transmit data including designated codes for events detected or created by an individual pacemaker. Individual pacemakers can be configured to issue a unique code corresponding to an event type and a location of the sending pacemaker.

Moreover, information communicated on the incoming channel can also include an event message from another LP signifying that the other LP has sensed a heartbeat or has delivered a pacing pulse, and identifies the location of the other pacemaker. For example, LP 102b may receive and relay an event message from LP 102a to the programmer 109. Similarly, information communicated on the outgoing channel can also include a message to another LP or pacemakers, or to the ICD 106, that the sending LP has sensed a heartbeat or has delivered a pacing pulse at the location of the sending pacemaker.

Referring again to FIGS. 1 and 2, the system 100 may comprise an ICD 106 in addition to one or more LPs 102a, 102b configured for implantation in electrical contact with a cardiac chamber and for performing cardiac rhythm management functions in combination with the implantable ICD 106. The implantable ICD 106 and the LPs 102a, 102b can be configured to perform i2i communication via conduction through body tissue and/or wireless transmission between transmitters and receivers in accordance with the embodiments discussed herein. Each of the LPs comprises at least two leadless electrodes 108 configured for delivering cardiac pacing pulses, sensing evoked and/or natural cardiac electrical signals, and optionally transmitting information to the co-implanted ICD 106.

Each of the LPs 102a, 102b can be configured for operation in a particular location and a particular functionality at manufacture and/or at programming by an external programmer. Bidirectional communication among the multiple LPs 102a, 102b can be arranged to communicate notification of a sensed heartbeat or delivered pacing pulse event and encoding type and location of the event to another implanted pacemaker or pacemakers. The LP 102a, 102b receiving the communication decodes the information and responds depending on location of the receiving LP and predetermined system functionality.

In some embodiments, the LPs 102a and 102b are configured to be implantable in any chamber of the heart, namely either atrium (RA, LA) or either ventricle (RV, LV). Furthermore, for dual-chamber configurations, multiple LPs may be co-implanted (e.g., one in the RA and one in the RV, one in the RV and one in the coronary sinus proximate the LV). Certain pacemaker parameters and functions depend on (or assume) knowledge of the chamber in (or on) which the pacemaker is implanted (and thus with which the LP is interacting; e.g., pacing and/or sensing). Some non-limiting examples include sensing sensitivity, an evoked response algorithm, use of AF suppression in a local chamber, blanking & refractory periods, etc. Accordingly, each LP needs to know an identity of the chamber in (or on) which the LP is implanted, and processes may be implemented to automatically identify a local chamber associated with each LP.

In an embodiment and also shown in FIG. 2, the primary battery 114 has positive terminal 140 and negative terminal 142. Current from the positive terminal 140 of primary battery 114 flows through an optional shunt 144 to an optional regulator circuit 146 to create a positive voltage supply 148 suitable for powering the remaining circuitry of the LP 102. The optional shunt 144 enables the optional battery current monitor 136 to provide the controller (e.g., processor) 112 with an indication of battery current drain and indirectly of device health. The illustrative power supply can be a primary battery 114.

Referring to FIG. 2, the LP 102a, 102b is shown as including an optional temperature sensor 152. The optional temperature sensor 152 can be any one of various types of well-known temperature sensors, or can be a future developed temperature sensor. The optional temperature sensor 152 can be used in various manners. For example, the optional temperature sensor 152 can be used to detect an activity level of the patient to adjust a pacing rate, i.e., for use in rate responsive pacing. When a person starts to exercise their core body temperature initially dips, and then after exercising for a prolonged period of time the person's core body temperature will eventually rise. Thereafter, when the person stops exercising their core body temperature will return to its baseline. Accordingly, the controller 112 can be configured to detect an activity level of a patient based on core blood temperature measurements obtained using the optional temperature sensor 152.

Referring to FIG. 2, the LP 102a, 102b is also shown as including an optional accelerometer 154, which can be hermetically contained within the housing 110. The optional accelerometer 154 can be any one of various types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. For example, the optional accelerometer 154 can be used to detect an activity level of the patient to adjust a pacing rate, i.e., for use in rate responsive pacing. It would also be possible to use outputs of both the optional accelerometer 154 and the optional temperature sensor 152 to monitor the activity level of a patient. Alternatively, or additionally, a patient's activity level can be monitored based on their heart rate, as detected from an electrogram (EGM) sensed using the electrodes 108, and/or sensed using a plethysmography signal obtained using a plethysmography sensor (not shown) or a heart sound sensor (not shown), but not limited thereto.

The controller 112 of the LP 102a can detect intrinsic atrial events from an EGM that is sensed by the LP 102a. Alternatively, or additionally, the controller 112 of the LP 102a can detect intrinsic atrial events from one or more signals sensed by the accelerometer 154 thereof and/or from a heart sounds signal sensed by a microphone (not shown) of the LP 102a. The controller 112 of the LP 102b can detect intrinsic ventricle events from an EGM that is sensed by the LP 102b. Alternatively, or additionally, the controller 112 of the LP 102b can detect intrinsic ventricular events from one or more signals sensed by the optional accelerometer 154 thereof and/or from a heart sounds signal sensed by a microphone (not shown) of the LP 102b. In certain embodiments, each of the LPs 102 includes only one of the optional temperature sensor 152 and the optional accelerometer 154.

In various embodiments, LP 102a, 102b can manage power consumption to draw limited power from the battery 114, thereby reducing device volume. Each circuit in the LP 102a, 102b can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

In some embodiments, the controller 112 of an LP 102 can access signals on the electrodes 108 and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller 112 can examine an output pulse waveform from another LP for usage as a signature for determining triggering information validity and, for a signature arriving within predetermined limits, activating delivery of a pacing pulse following a predetermined delay of zero or more milliseconds.

Instead of or in addition to the LP 102 utilizing conductive communication to communicate with another LP, another type of IMD, and/or an external device (e.g., 109), the LP 102 can include an RF or inductive transceiver (not shown) that is coupled to the controller 112, and the RF or inductive transceiver can be coupled to an antenna or inductive coil (not shown), to thereby enable the LP 102 to utilize RF communication and/or inductive communication to communicate with another LP, another type of IMD, and/or an external device (e.g., 109). In other words, the communication performed by the LP 102 can be conductive communication, RF communication, or inductive communication, or any combination thereof.

FIG. 3 shows an LP 102a, 102b. The LP can include a hermetic housing 202 with electrodes 108a and 108b disposed thereon. As shown, electrode 108a can be separated from but surrounded partially by a fixation mechanism 205, and the electrode 108b can be disposed on the housing 202. The fixation mechanism 205 can be a fixation helix, a plurality of hooks, barbs, or other attaching features configured to attach the pacemaker to tissue, such as heart tissue. The electrodes 108a and 108b are examples of the electrodes 108 shown in and discussed above with reference to FIG. 2.

The housing 202 can also include an electronics compartment 210 within the housing that contains the electronic components necessary for operation of the pacemaker, including, e.g., a pulse generator, receiver, a battery, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator 208 disposed on the conductive material to separate electrodes 108a and 108b. The insulator 208 can be an insulative coating on a portion of the housing 202 between the electrodes 108a and 108b, and can comprise materials, such as silicone, polyurethane, parylene, or another biocompatible electrical insulator commonly used for implantable medical devices. In the embodiment of FIG. 2, a single insulator 208 is disposed along the portion of the housing 202 between electrodes 108a and 108b. In some embodiments, the housing 202 itself can comprise an insulator instead of a conductor, such as an alumina ceramic or other similar materials, and the electrodes can be disposed upon the housing 202.

As shown in FIG. 3, the pacemaker can further include a header assembly 212 to isolate electrodes 108a and 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a and 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In FIG. 3, electrode 108a can be a pace/sense electrode and electrode 108b can be a return electrode. The electrode 108b can be a portion of the conductive housing 202 that does not include an insulator 208.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in FIG. 3) into contact with stimulable tissue. Electrode 108b can serve as an indifferent electrode for sensing and pacing. The fixation mechanism may be coated partially or in full for electrical insulation, and a steroid-eluting matrix may be included on or near the device to minimize fibrotic reaction, as is known in conventional pacing electrode-leads.

### Implant-to-Implant Event Messaging

LPs 102a and 102b can utilize implant-to-implant (i2i) communication through event messages to coordinate operation with one another in various manners. The terms i2i communication, i2i event messages, and i2i event markers are used interchangeably herein to refer to event related messages and IMD/IMD operation related messages transmitted from an implanted device and directed to another implanted device (although external devices, e.g., a programmer, may also receive i2i event messages). In certain embodiments, LP 102a and LP 102b operate as two independent leadless pacers maintaining beat-to-beat dual-chamber functionality via a "Master/Slave" operational configuration. For descriptive purposes, the ventricular LP 102b shall be referred to as "vLP" and the atrial LP 102a shall be referred to as "aLP". The LP 102 that is designated as the master device (e.g. vLP 102b) may implement all or most dual-chamber diagnostic and therapy determination algorithms. For purposes of the following illustration, it is assumed that the vLP 102b is a "master" device, while the aLP 102a is a "slave" device. Alternatively, the aLP 102a may be designated as the master device, while the vLP 102b may be designated as the slave device. The master device orchestrates most or all decision-making and timing determinations (including, for example, rate-response changes).

Herein, methods are provided for coordinating operation between first and second LPs 102a, 102b configured to be implanted in (or on) first and second chambers of the heart. In such methods, an event marker is transmitted using conductive communication through electrodes 108 located along a housing 110 of the first LP 102a, 102b, wherein the event marker is indicative of one of a local paced or sensed event. The method detects, over a sensing channel, the event marker at the second LP 102b, 102a. The event marker is identified at the second LP 102b, 102a based on a predetermined pattern configured to indicate that an event of interest has occurred in a remote chamber. In response to the identifying operation, a related action is initiated in the second LP 102b, 102a.

FIG. 4 is a timing diagram 400 demonstrating one example of an i2i communication for a paced event. The i2i communication may be transmitted, for example, from LP 102a to LP 102b. As shown in FIG. 4, in this embodiment, an i2i transmission 402 is sent prior to delivery of a pace pulse 404 by the transmitting LP (e.g., LP 102a). This enables the receiving LP (e.g., LP 102b) to prepare for the remote delivery of the pace pulse. The i2i transmission 402 includes an envelope 406 that may include one or more individual pulses. For example, in this embodiment, envelope 406 includes a low frequency pulse 408 followed by a high frequency pulse train 410. Low frequency pulse 408 lasts for a period T_{i2iLF}, and high frequency pulse train 410 lasts for a period T_{i2iHF}. The end of low frequency pulse 408 and the beginning of high frequency pulse train 410 are separated by a gap period, T_{i2iGap}.

As shown in FIG. 4, the i2i transmission 402 lasts for a period Ti2iP, and pace pulse 404 lasts for a period Tpace. The end of i2i transmission 402 and the beginning of pace pulse 404 are separated by a delay period, TdelayP. The delay period may be, for example, between approximately 0.0 and 10.0 milliseconds (msec), particularly between approximately 0.1 msec and 2.0 msec, and more particularly approximately 1.0 msec. The term approximately, as used herein, means +/- 10% of a specified value.

FIG. 5 is a timing diagram 500 demonstrating one example of an i2i communication for a sensed event. The i2i communication may be transmitted, for example, from LP 102a to LP 102b. As shown in FIG. 5, in this embodiment, the transmitting LP (e.g., LP 102a) detects the sensed event when a sensed intrinsic activation 502 crosses a sense threshold 504. A predetermined delay period, T_{delayS}, after the detection, the transmitting LP transmits an i2i transmission 506 that lasts a predetermined period T_{i2iS}. The delay period may be, for example, between approximately 0.0 and 10.0 milliseconds (ms), particularly between approximately 0.1 msec and 2.0 msec, and more particularly approximately 1.0 msec.

As with i2i transmission 402, i2i transmission 506 may include an envelope that may include one or more individual pulses. For example, similar to envelope 406, the envelope of i2i transmission 506 may include a low frequency pulse followed by a high frequency pulse train.

Optionally, wherein the first LP 102a is located in an atrium and the second LP 102b is located in a ventricle, the first LP 102a produces an AS or AP event marker to indicate that an atrial sensed (AS) event has occurred or an atrial paced (AP) event has occurred or will occur in the immediate future. For example, the AS and AP event markers may be transmitted following the corresponding AS or AP event. Alternatively, the first LP 102a may transmit the AP event marker slightly prior to delivering an atrial pacing pulse. In certain embodiments, wherein the first LP 102a is located in an atrium and the second LP 102b is located in a ventricle, the second LP 102b initiates an atrioventricular (AV) interval in response to receiving an AS or AP event marker from the first LP 102a. It is also possible that the second LP 102b can initiate an AV interval in response to receiving an AP event marker from the first LP 102a, and the second LP 102b can initiate a PV interval in response to receiving an AS event marker from the first LP 102a, where the duration of AV interval may be greater than the duration of the PV interval by about 25 msec, but not limited thereto. Additionally, the second LP 102b can initiate a post atrial ventricular blanking (PAVB) interval after receiving an AP event marker from the first LP 102a.

In accordance with some embodiments, communication and synchronization between the aLP 102a and vLP 102b is implemented via conducted communication of markers/commands in the event messages (per i2i communication protocol). As explained above, conducted communication represents event messages transmitted from the sensing/pacing electrodes 108 at frequencies outside the RF or Wi-Fi frequency range. Alternatively, the event messages may be conveyed over communication channels operating in the RF or Wi-Fi frequency range. The figures and corresponding description below illustrate non-limiting examples of markers that may be transmitted in event messages. The figures and corresponding description below also include the description of the markers and examples of results that occur in the LP 102 that receives the event message. Table 1 represents exemplary event markers sent from the aLP 102a to the vLP 102b, while Table 2 represents exemplary event markers sent from the vLP 102b to the aLP 102a. In the master/slave configuration, AS event markers are sent from the aLP 102a each time that an atrial event is sensed outside of the post ventricular atrial blanking (PVAB) interval or some other alternatively-defined atrial blanking period. The AP event markers are sent from the aLP 102a each time that the aLP 102a delivers a pacing pulse in the atrium. The aLP 102a may restrict transmission of AS markers, whereby the aLP 102a transmits AS event markers when atrial events are sensed both outside of the PVAB interval and outside the post ventricular atrial refractory period (PVARP) or some other alternatively-defined atrial refractory period. Alternatively, the aLP 102a may not restrict transmission of AS event markers based on the PVARP, but instead transmit the AS event marker every time an atrial event is sensed.

**Table 1**

| "A2V" Markers / Commands (*i.e*., from aLP to vLP) | | |
|---|---|---|
| *Marker* | *Description* | *Result in vLP* |
| AS | Notification of a sensed event in atrium (if not in PVAB or PVARP) | • Initiate PV interval (if not in PVAB or PVARP) |
| AP | Notification of a paced event in atrium | • Initiate PAVB |
| | | • Initiate AV interval (if not in PVAB or PVARP) |

As shown in Table 1, when an aLP 102a transmits an event message that includes an atrial sensed (AS) event marker (indicating that the aLP sensed an intrinsic atrial event), the vLP 102b initiates a PV interval timer (also known as an AS-VP interval timer). If the aLP 102a transmits an AS event marker for all sensed events, then the vLP 102b would preferably first determine that a PVAB or PVARP interval is not active before initiating the PV interval timer. If however the aLP 102a transmits an AS event marker only when an intrinsic signal is sensed outside of a PVAB or PVARP interval, then the vLP 102b could initiate the PV interval timer upon receiving an AS event marker without first checking the PVAB or PVARP status. When the aLP 102a transmits an atrial paced (AP) event marker (indicating that the aLP 102a delivered or is about to deliver a pace pulse to the atrium), the vLP 102b initiates a PAVB timer and an AV interval timer (also known as an AP-VP interval timer), provided that a PVAB or PVARP interval is not active. The vLP 102b may also blank its sense amplifiers to prevent possible crosstalk sensing of the remote pace pulse delivered by the aLP 102a.

**Table 2**

| "V2A" Markers / Commands (*i.e.,* from vLP to aLP) | | |
|---|---|---|
| *Marker* | *Description* | *Result in aLP* |
| VS | Notification of a sensed event in ventricle | • Initiate PVAB |
| | | • Initiate PVARP |
| VP | Notification of a paced event in ventricle | • Initiate PVAB |
| | | • Initiate PVARP |
| AP | Command to deliver immediate pace pulse in atrium | • Deliver immediate pace pulse to atrium |

As shown in Table 2, when the vLP 102b senses a ventricular event, the vLP 102b transmits an event message including a ventricular sensed (VS) event marker, in response to which the aLP 102a may initiate a PVARP interval timer. When the vLP 102b delivers or is about to deliver a pace pulse in the ventricle, the vLP 102b transmits ventricular paced (VP) event marker. When the aLP 102a receives the VP event marker, the aLP 102a initiates the PVAB interval timer and also the PVARP interval timer. The aLP 102a may also blank its sense amplifiers to prevent possible crosstalk sensing of the remote pace pulse delivered by the vLP 102b. In accordance with certain embodiments, the vLP 102b may also transmit an event message containing an AP command marker to command the aLP 102a to deliver an immediate pacing pulse in the atrium upon receipt of the command without delay.

The foregoing event markers are examples of a subset of markers that may be used to enable the aLP 102a and vLP 102b to maintain full dual-chamber functionality. In one embodiment, the vLP 102b may perform all dual-chamber algorithms, while the aLP 102a may perform atrial-based hardware-related functions, such as PVAB, implemented locally within the aLP 102a. In this embodiment, the aLP 102a is effectively treated as a remote 'wireless' atrial pace/sense electrode. In another embodiment, the vLP 102b may perform most but not all dual-chamber algorithms, while the aLP 102a may perform a subset of diagnostic and therapeutic algorithms. In an alternative embodiment, vLP 102b and aLP 102a may equally perform diagnostic and therapeutic algorithms. In certain embodiments, decision responsibilities may be partitioned separately to one of the aLP 102a or vLP 102b. In other embodiments, decision responsibilities may involve joint inputs and responsibilities.

In an embodiment, ventricular-based pace and sense functionalities are not dependent on any i2i communication, in order to provide safer therapy. For example, in the event that LP to LP (i2i) communication is lost (prolonged or transient), the system 100 may automatically revert to safe ventricular-based pace/sense functionalities as the vLP 102b is running all of the necessary algorithms to independently achieve these functionalities. For example, the vLP 102b may revert to a VVI mode (also known as VVI operation) during which the vLP 102b does not depend on i2i communication to perform ventricular pace/sense activities. For another example, the aLP 102a may revert to AAI mode (also known as AAI operation) during which the aLP does not depend on i2i communication to perform atrial pace/sense activities. In accordance with an embodiment, once i2i communication is restored, the system 100 can automatically resume dual-chamber functionalities. As will be described in additional detail below, it is also possible that the aLP 102a and the vLP 102b can be configured to operate at least some of the time in an AAI + VVI mode (which can also be referred to as AAI + VVI operation) during which the aLP and the vLP purposely abstain from communicating with one another using i2i communication to conserve their energy and thereby increase their longevity.

Messages that are transmitted between LPs 102 (e.g., the aLP 102a and the vLP 102b) can be referred to herein generally as i2i messages, since they are implant-to-implant messages. As noted above, such messages can include event markers that enable one LP to inform the other LP of a paced event or a sensed event. For example, in certain embodiments, whenever the aLP 102a senses an atrial event or paces the right atrium, the aLP 102a will transmit an i2i message to the vLP 102b to inform the vLP 102b of the sensed or paced event in the atrium. In response to receiving such an i2i message, the vLP 102b may start one or more timers that enable the vLP 102b to sense or pace in the right ventricle. Similarly, the vLP 102b may transmit an i2i message to the aLP 102a whenever the vLP 102b senses a ventricular event or paces the right ventricle.

The i2i messages that are sent between LPs 102 may be relatively short messages that simply allow a first LP to inform a second LP of an event that was sensed by the first LP or caused (paced) by the first LP, and vice versa. Such i2i messages can be referred to herein as event marker i2i messages, or more succinctly as event i2i messages, or even more succinctly as event messages. The i2i messages that are sent between LPs 102, in certain instances, can be extended i2i messages that include (in addition to an event marker) an extension. In certain embodiments, an extended i2i message includes an event marker (e.g., 9 bits), followed by an extension indicator (e.g., 2 bits), followed by an extended message payload portion (e.g., 17 bits), followed by a cyclic redundancy check (CRC) code (e.g., 6 bits) or some other type of error detection and correction code. In other words, some i2i messages can include a message payload in addition to an event marker.

In certain embodiments, whenever an i2i message is sent by an LP (or other type of IMD, such as an ICD), the i2i message will include an extension indicator so that the receiving LP knows whether or not the i2i message it receives includes an extension portion. In such embodiments, even a relatively short event i2i message will include an extension indicator. The extension indicator (e.g., 2 bits) is used by the LP (or other IMD) sending the i2i message to indicate, to the LP receiving the i2i message, whether or not the i2i message is an extended i2i message. In certain embodiments, if the LP receiving an i2i message determines based on the extension indicator bits that the received i2i message is not an extended i2i message, then the LP receiving the i2i message can ignore any bits that follow the extension bits. In such a case, the LP receiving the i2i message only decodes the event marker. On the other hand, if the LP receiving an i2i message determines based on the extension indicator bits that the received i2i message is an extended i2i message, then the LP receiving the i2i message will also decode the bits that follow the extension bits, and determine based on a CRC code (or other type of error detection and correction code), whether or not the i2i message is a valid message. If the extended i2i message is a valid i2i message, then the LP receiving the extended i2i message will as appropriate modify its operation, update one or more parameters, and/or the like, based on information included in the extended i2i message. In certain embodiments, event i2i messages that are not extended i2i messages do not include any error detection and correction code.

In an extended i2i message, the event marker bits and the extension indicator bits are located, respectively, in an event marker field and an extension indicator field of an i2i message packet. In certain embodiments, the extended portion (that follows the event marker bits and the extension indicator bits) includes message bits (in a message field) and rate indicator bits (in a rate indicator field), which are parts of the message payload. The message payload can alternatively, or additionally, include other types of fields, such as an acknowledgement field that is used in certain situations for one LP to acknowledge reception of an i2i message from another LP of certain (e.g., critical) types of message.

More generally, various types of information may be included within the message payload of an extended i2i message. For example, the message payload can include a pacing rate indicator that enables one LP to inform another LP of a pacing rate. Assume that a multi-chamber LP system provides rate responsive pacing, wherein a pacing rate is adjusted in dependence on a patient's physical activity as detected, e.g., using an accelerometer, temperature sensor, and/or other type of sensor of an LP. In such a multi-chamber LP system, the vLP 102b may inform the aLP 102a of the rate at which the patient's heart should be paced so that the aLP 102a and vLP 102b can perform synchronized pacing. To achieve this, the vLP 102b can send a pacing rate indicator to the aLP 102a in the message payload of an i2i message. The pacing rate indicator can, e.g., be a value indicating a pacing rate value (e.g., 80 bpm), a code that the aLP 102a that can look up (e.g., in a stored look up table) and corresponds to a pacing rate value, or a value that the aLP 102a feeds into an equation to determine the pacing rate, but is not limited thereto. Alternatively, the pacing rate indicator can be beat-to-beat interval value (e.g., 0.75 seconds), a code that the aLP 102a can look up and corresponds to a beat-to-beat interval value, or a value that the aLP 102a feeds into an equation to determine the beat-to-beat interval, but is not limited thereto. Other variations are also possible and within the scope of the embodiments described herein. The message payload can alternatively or additionally include, for example, a recommended replacement time (RRT) indicator, an automatic mode switch (AMS) entry indicator, an AMS exit indicator, a magnet entry indicator, or a magnet exit indicator. For still another example, the message payload of an i2i message transmitted from an aLP 102a to a vLP 102b can include atrial interval information, such as an average atrial rate interval or a Filtered Atrial Rate Interval (FARI), based upon which the vLP can set a rate responsive refractory period duration.

FIG. 6 will now be used to describe example details of the timing circuitry 160 introduced above in the discussion of FIG. 2, in accordance with an embodiment of the present technology. Referring to FIG. 6, the timing circuitry 160 of the LP 102 is shown as including one or more clock signal generators 602 configured to produce a low frequency (LF) clock signal and a high frequency (HF) clock signal having a frequency that is at least one order of magnitude (i.e., at least 10 times) faster than the LF clock signal. In some embodiments, the frequency of the HF clock signal is at least 10 times, preferably at least 25 times, more preferably at least 50, and most preferably at least 100 times, such as at least 100 times faster or higher than the frequency of the LF clock signal. In accordance with certain embodiments, the LF clock signal has a frequency within the range of 10 Hz to 500 Hz, and the HF clock signal has a frequency within the range of 1 kHz to 50 kHz. For example, if the LF clock signal is 128 Hz, then the HF clock signal is at least 1.28 kHz. In a specific example embodiment, the LF clock signal is 128 Hz and the HF clock signal is ~32 kHz, in which case the HF clock signal is at least two orders of magnitude (i.e., at least 100 times) faster than the LF clock signal. These are just example ranges and values for the LF and HF clock signals, which examples are not intended to be limiting. The LF clock signal is shown as being provided to a low resolution counter 604, and the HF clock signal is shown as being provided to a high resolution counter 606. The low resolution counter 604 and the high resolution counter 606 can be part of the timing circuitry 160 or can be separate from the timing circuitry 160, depending upon the specific implementation. Regardless of the specific implementation, the low resolution counter 604 should receive the LF clock signal, and the high resolution counter 606 should receive the HF clock signal. In accordance with certain embodiments, the low resolution counter 604 counts rising or falling edges of the LF clock signal (or more generally, a first clock signal) having a first frequency, and the high resolution counter 606 counts rising or falling edges of the HF clock signal (or more generally, a second clock signal) having a second frequency that is at least 10 times faster (and may be at least 100 time faster) than the first frequency. The outputs of the low resolution counter 604 and the high resolution counter 606 are shown as being provided to the controller 112 (in FIG. 2). As will be described in additional detail further below, the counters 604, 606 are used to determine count values that are stored in memory 162 during a period of time that a first one of the LPs (aka a first LP) successfully receives event messages from a second one of the LPs (aka a second LP). The controller 112 determines a compensation offset (indicative of a drift between respective timing circuitry 160 of the LPs 102a and 102b) based on one or more of the stored count values. As will be described in additional detail further below, the compensation offset, after it is determined and stored, is used to compensate for the drift between the timing circuitry 160 of the first LP and the timing circuitry 160 of the second LP so that the AV synchrony is maintained during a further period of time that the first LP does not receive event messages from the second LP. The drift between the timing circuitry 160 of the first LP and the timing circuitry 160 of the second LP can also be referred to herein as the drift between the timing circuitry 160 of the first LP and the timing circuitry 160 of the second LP.

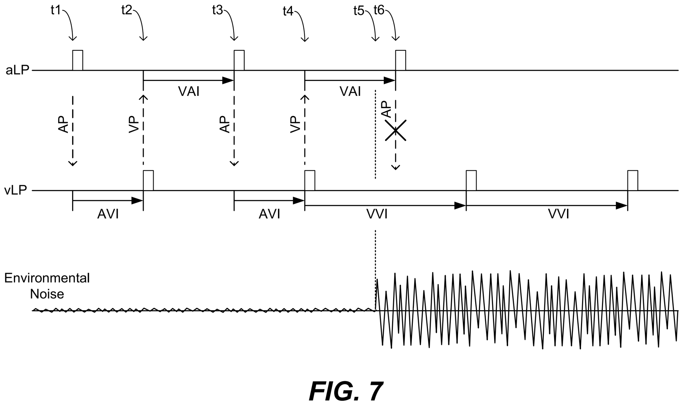

Reference is now made to FIG. 7, which is a timing diagram illustrating operations of the aLP 102a and vLP 102b, and i2i communications therebetween. The upper waveform in FIG. 7 is used to describe operations of the aLP 102a, the middle waveform in FIG. 7 is used to describe operations of the vLP 102b, and the lower waveform in FIG. 7 is used to illustrate a level of environmental noise which affects that ability of the LPs 102a and 102b to successfully communicate with one another using i2i communication.

Referring to FIG. 7, at time t1 the aLP 102a causes a paced atrial event (aka an atrial paced event, or an AP), and sends an i2i message to the vLP 102b to inform the vLP of the AP. In certain embodiments, the aLP sends the i2i message to the vLP 102b to inform the vLP 102b of the AP, just prior to the aLP 102a causing the paced atrial event (aka an atrial paced event, or an AP). The vLP 102b, in response to being informed of the AP, initiates an atrioventricular (AV) interval (aka AVI).

At time t2 the AV interval (aka AVI) expires and the vLP 102b causes a paced ventricular event (aka a ventricular paced event, or a VP), and the vLP 102b sends an i2i message to the aLP 102a to inform the aLP 102a of the VP. In certain embodiments, the vLP 102b sends the i2i message to the aLP 102a to inform the aLP 102a of the VP, just prior to the vLP 102b causing the paced ventricular event (aka a ventricular paced event, or a VP). The aLP 102a, in response to being informed of the VP, initiates a VA interval (aka VAI).

At time t3, the VA interval (aka VAI) expires and the aLP 102a causes an AP, and sends an i2i message to the vLP 102b to inform the vLP of the AP. The vLP 102b, in response to being informed of the AP, initiates an AVI. As noted above, in certain embodiments, the aLP sends the i2i message to the vLP 102b to inform the vLP 102b of the AP, just prior to the aLP 102a causing the paced atrial event (aka an atrial paced event, or an AP).

At time t4 the AV interval (aka AVI) expires and the vLP 102b causes a paced ventricular event (aka a ventricular paced event, or a VP), and the vLP 102b sends an i2i message to the aLP 102a to inform the aLP 102a of the VP. As noted above, in certain embodiments, the vLP 102b sends the i2i message to the aLP 102a to inform the aLP 102a of the VP, just prior to the vLP 102b causing the paced ventricular event (aka a ventricular paced event, or a VP). The aLP 102a, in response to being informed of the VP, initiates a VA interval (aka VAI).

In FIG. 7, there is substantially no environmental noise during the times t1, t2, t3, and t4. However, starting at time t5 there is significant environmental noise that causes the LPs 102a and 102b to be unable to successfully communicate with one another using i2i communication. In certain dual chamber LP systems, when the LPs 102a and 102b are unable to successfully communicate with one another using i2i communication, they transition from an operational mode that provides for dual chamber pacing, such as DOO mode, to an operational mode that provides for single chamber pacing, such as VOO mode, where only the ventricular chamber (e.g., the right ventricle) is paced.

As noted above, when the LPs 102a and 102b are unable to successfully perform i2i communication, each LP can attempt to rely on its own respectively timing circuitry (e.g., its own respective one or more clock signal generators 602) to continue to collectively operate in the DOO mode, during which dual chamber pacing is performed. More specifically, the aLP 102a can time its delivery of atrial pacing stimulation using its own timing circuitry such that the atrial chamber (in or on which the aLP 102a is implanted) is paced in accordance with an AA interval corresponding to a base pacing rate, and the vLP 102b can time its deliver of ventricular pacing stimulation using its own timing circuitry such that the ventricular chamber (in or on which the vLP is implanted) is paced in accordance with a VV interval corresponding to the base pacing rate. While this type of dual chamber pacing without i2i communication may work in theory, that may not be the case in the real world due to drift between the timing circuitry 160 of the aLP 102a and the timing circuitry 160 of the vLP 102b. More specifically, because the aLP 102a and the vLP 102b include their own respective timing circuitry 160 (e.g., including their own respective one or more clock signal generators 602) which drift relative to one another of time, if the aLP 102a and the vLP 102b respectively deliver atrial pacing stimulation and ventricular pacing stimulation independent of one another, the multi-chamber LP system 100 may not reliably maintain AV synchrony. This may result in the vLP 102b delivering pacing stimulation to the ventricular chamber (in or on which the vLP 102b is implanted) at a time that is significantly earlier than (or significantly later than) a specified AVI following when the aLP 102a delivered atrial stimulation to the atrial chamber (in or on which the aLP 102a is implanted), which is undesirable and may lead to poor patient outcomes. Similarly, this may result in the aLP 102a delivering pacing stimulation to the atrial chamber (in or on which the aLP 102a is implanted) at a time that is significantly earlier than (or significantly later than) a specified VAI following when the vLP 102b delivered ventricular stimulation to the ventricular chamber (in or on which the vLP 102b is implanted), which is also undesirable and may lead to poor patient outcomes. A base pacing rate is a programmed pacing rate that ensures that a patient's heart maintains a minimum heart rate. A base pacing rate interval is the beat-to-beat interval that corresponds to the base pacing rate. For example, if a base pacing rate is set to 60 bpm, then the base pacing rate interval is 1000 msec. For another example, if the base pacing rate is set to 70 bpm, then the base pacing rate interval is 857 msec. The base pacing rate (and/or the base pacing rate interval) can be set by default or can be set by a physician based on a patient's specific needs and/or medical condition. The base pacing rate is often within the range of 50 to 70 beats per minute (bpm), with a typically base pacing rate being 60 bpm. In certain embodiments, the base pacing rate is rate responsive, meaning it increases with increases in patient activity which can be detected using a temperature sensor and/or an accelerometer, as is known in the art. If the base pacing rate is rate responsive, then the base pacing rate interval is also rate responsive, in which case the base pacing rate interval decreases with increases in patient activity.

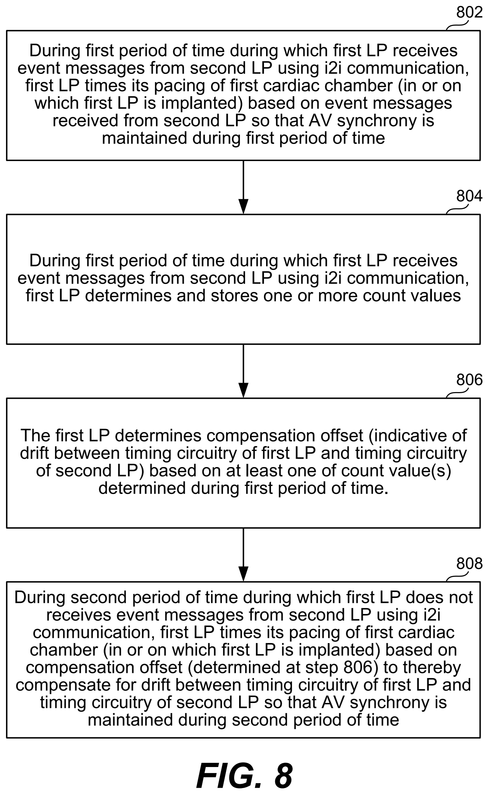

Embodiments of the present technology described below compensate for the drift between the timing circuitry 160 of the vLP 102b and the timing circuitry 160 of the aLP 102a so that AV synchrony is maintained during a period of time during which the vLP 120b does not receive i2i messages from the aLP 102a. More generally, embodiments of the present technology described below compensate for the drift between timing circuitry 160 of a first LP and timing circuitry 160 of a second LP so that the AV synchrony is maintained during a period of time during which the first LP does not receive i2i messages from the second LP. As will be described in additional detail below with reference to the high level flow diagram of FIG. 8, in accordance with certain embodiments the aforementioned drift is compensated for by at least one of the LPs (and possibly both of the LPs) by a first one of the LPs (aka a first LP) determining and storing a compensation offset based on one or more count values determined and stored during a period of time that the first LP successfully receives event messages from a second one of the LPs (aka a second LP), and thereafter using the compensation offset during a further period of time that the first LP does not receive event messages from the second LP. As can be appreciated from the above discussion of FIG. 7, a reason that the first LP may not receive event messages from the second LP during a period of time may be due to extrinsic interference. Another potential reason that the first LP may not receive event messages from the second LP during a period of time may be due to the second LP abstaining from transmitting event messages to conserve its energy. Still further reasons that the first LP may not receive event messages from the second LP during a period of time may be due to the first LP disabling its receiver, or due to there being instability in a communication channel between the LPs, just to name a few.

The high level flow diagram of FIG. 8 is now used to summarize methods for use by a first LP configured to communicate with a second LP using i2i communication, wherein the first LP is configured to be implanted in or on a first cardiac chamber and to perform pacing of the first cardiac chamber, and wherein the second LP is configured to be implanted in or on a second cardiac chamber and to perform pacing of the second cardiac chamber. Such methods can be used to compensate for the drift between timing circuitry 160 of the first LP and timing circuitry 160 of the second LP so that the AV synchrony is maintained during a period of time during which the first LP does not receive i2i messages from the second LP. The steps of the methods described with reference to FIG. 8 can be performed by a controller (e.g., 112) of the first LP (e.g., 102a, 102b). The count values that are stored in the steps of FIG. 8 can be stored in memory (e.g., 162) of the first LP.

Referring to FIG. 8, step 802 involves, during a first period of time during which the first LP receives event messages from the second LP using i2i communication, the first LP timing its pacing of the first cardiac chamber based on the event messages received from the second LP using i2i communication so that AV synchrony is maintained during the first period of time. For example, if the first LP is configured to be implanted in or on a ventricular chamber (e.g., the right ventricle) or proximate the LBB and to perform pacing of the ventricular chamber, and the second LP is configured to be implanted in or on an atrial chamber (e.g., the right atrium) and to perform pacing of the atrial chamber, then in order to maintain AV synchrony the first LP (e.g., the vLP 102b) can pace the first cardiac chamber (which in this case the ventricular chamber) at an AV interval following the first LP receiving an event message from the second LP (e.g., the aLP 102a) informing first LP that the second LP delivered or is about to deliver pacing stimulation to the second cardiac chamber (which in this case in the atrial chamber). For another example, if the first LP is configured to be implanted in or on an atrial chamber (e.g., the right atrium) and to perform pacing of the atrial chamber, and the second LP is configured to be implanted in or on a ventricular chamber (e.g., the right ventricle) or proximate the LBB and to perform pacing of the ventricular chamber, then in order to maintain AV synchrony the first LP (e.g., the aLP 102a) can pace the first cardiac chamber (which in this case the atrial chamber) at a VA interval following the first LP receiving an event message from the second LP (e.g., the vLP 102b) informing first LP that the second LP delivered or is about to deliver pacing stimulation to the second cardiac chamber (which in this case in the ventricular chamber).

Still referring to FIG. 8, step 804 involves, also during the first period of time during which the first LP receives the event messages from the second LP using i2i communication, the first LP determining and storing one or more count values.

In accordance with certain embodiments, the first LP determines, at step 804, a measured count value corresponding to a duration between when the first LP delivers pacing stimulation to the first cardiac chamber and when the first LP receives an event message, from the second LP (informing first LP that the second LP delivered or is about to deliver pacing stimulation to the second cardiac chamber). For example, if the first LP is the vLP 102b configured to be implanted in or on a ventricular chamber (e.g., the right ventricle) or proximate the LBB and to perform pacing of the ventricular chamber and the second LP is the aLP 102a configured to be implanted in or on an atrial chamber (e.g., the right atrium), then the measured count value may correspond to a duration between when the vLP 102b delivers pacing stimulation to the ventricular chamber and when the vLP 102b receives an event message from the aLP 102a (informing vLP 102b that the aLP 102a delivered or is about to deliver pacing stimulation to the atrial chamber). Alternatively, if the first LP is the aLP 102a configured to be implanted in or on an atrial chamber (e.g., the right atrium) and to perform pacing of the atrial chamber and the second LP is the vLP 102b configured to be implanted in or on an ventricular chamber (e.g., the right ventricle) or proximate the LBB, then the measured count value may correspond to a duration between when the aLP 102a delivers pacing stimulation to the atrial chamber and when the aLP 102a receives an event message from the vLP 102b (informing aLP 102a that the vLP 102b delivered or is about to deliver pacing stimulation to the ventricular chamber).

In an embodiment, step 804 is performed while the first LP and the second LP are collectively operating in a DOO mode or a DOOR mode, during which there is dual chamber pacing (of the atrial and ventricular chambers in or on which the LPs are implanted), and during which sensing is turned off, and response to sensing is turned off.

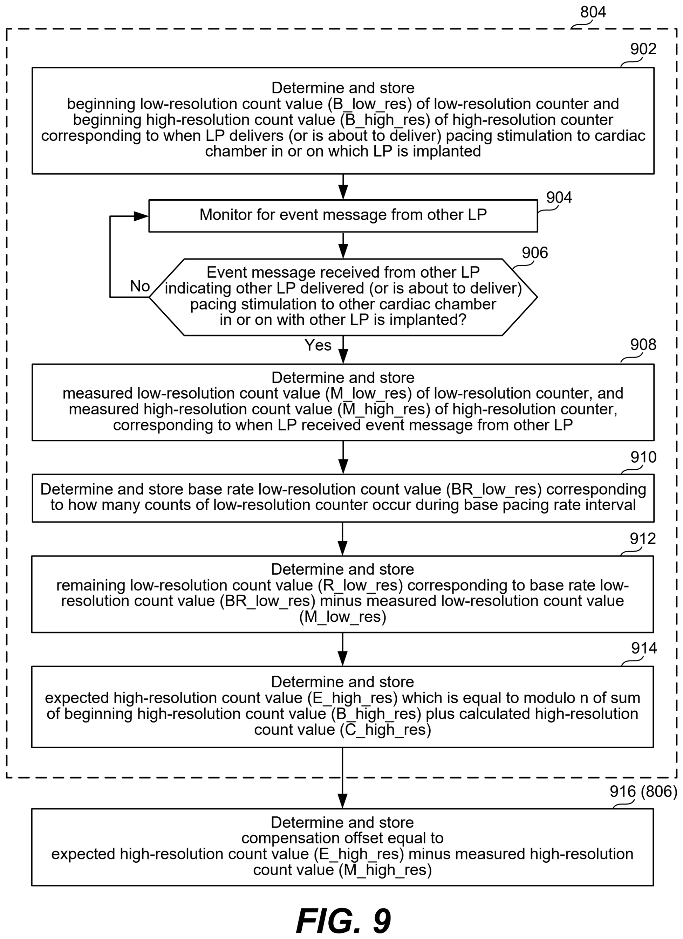

Step 806 involves the first LP determining a compensation offset indicative of a drift between timing circuitry 160 of the first LP and timing circuitry 160 of the second LP. In accordance with certain embodiments, the first LP determines, at step 806, the compensation offset by subtracting the measured count value (determined at step 804) from an expected count value. In certain such embodiments, the expected count value corresponds to what the measured count value would be if there was no drift between the timing circuitry of the first LP and the timing circuitry of the second LP. For an example, assume the first LP is the vLP 102b, the second LP is the aLP 102a, a counter of the vLP 102b has a frequency of 32 kHz, and a programmed ventricular-atrial interval (VAI) is 880 msec (i.e., 0.88 sec), then the expected count value = 32,000 counts/seconds * 0.88 seconds = 28,160 counts. For another example, assume the first LP is the aLP 102a, the second LP is the vLP 102b, a counter of the aLP 102a has a frequency of 32 kHz, and a programmed atrioventricular interval (AVI) is 120 msec (i.e., 0.12 sec), then the expected count value = 32,000 counts/seconds * 0.12 seconds = 3,840 counts. Such an expected count value can be determined by the first LP prior to the first period of time, during the first period of time, or after the first period of time, depending upon the specific implementation. Additional details of step 806, according to certain embodiments of the present technology, are described below with reference to FIG. 9.

Still referring to FIG. 8, step 808 involves, during a second period of time during which the first LP does not receive event messages from the second LP, the first LP timing its pacing of the first cardiac chamber, based on the compensation offset that the first LP determined at step 806, to thereby compensate for the drift between the timing circuitry of the first LP and the timing circuitry of the second LP so that the AV synchrony is maintained during the second period of time.

Referring briefly back to step 806, the compensation offset (which is used at step 808) could have been determined during the first period of time (during which the first LP receives the event messages from the second LP using i2i communication), or during the second period of time (during which the first LP does not receive event messages from the second LP) based on one or more of the count values that the first LP had stored during the first period of time.

In accordance with certain embodiments, if the first LP is configured to be implanted in or on a ventricular chamber (e.g., the right ventricle) or proximate the LBB and to perform pacing of the ventricular chamber, then step 808 includes the first LP (e.g., the vLP 102b) determining an expected time at which the second LP (e.g., the aLP 102a) delivers pacing stimulation to the second cardiac chamber (e.g., the right atrium). In such an embodiment, step 808 also includes, at a compensated AV interval following the expected time at which the second LP (e.g., the aLP 102a) delivers pacing stimulation to the second cardiac chamber (e.g., the right atrium), the first LP (e.g., 102b) delivering pacing stimulation to the first cardiac chamber (e.g., the right ventricle).

The compensated AV interval can be based on the compensation offset and a programmed AV interval. For example, the compensated AV interval can be equal to the programmed AV interval minus the compensation offset.

Alternatively, if the first LP is configured to be implanted in or on an atrial chamber (e.g., the right atrium) and to perform pacing of the atrial chamber, then step 808 includes the first LP (e.g., the aLP 102a) determining an expected time at which the second LP (e.g., the vLP 102b) delivers pacing stimulation to the second cardiac chamber. In such an embodiment, step 808 also includes at a compensated VA interval following the expected time at which the second LP (e.g., the vLP 102b) delivers pacing stimulation to the second cardiac chamber (e.g., the right ventricle), the first LP (e.g., the aLP 102a) delivering pacing stimulation to the first cardiac chamber (e.g., the right atrium).

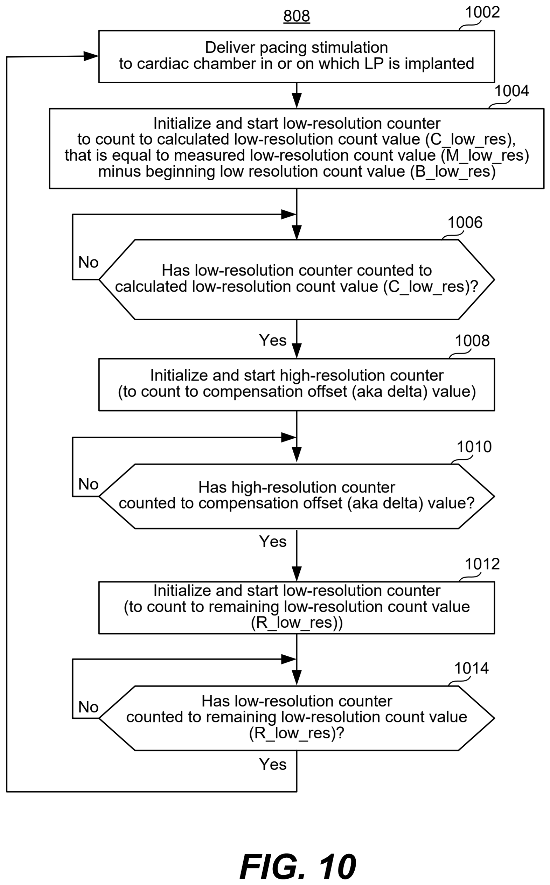

The compensated VA interval can be based on the compensation offset and a programmed VA interval. For example, the compensated VA interval can be equal to the programmed VA interval minus the compensation offset. Additional details of step 808, according to certain embodiments of the present technology, are described below with reference to FIG. 10.

Referring now to FIG. 9, many of the steps shown in and described with reference to FIG. 9 can be considered sub-steps of step 804, in accordance with certain embodiments of the present technology. Such steps are performed during the first period of time, during which the first LP (which can also be referred to more succinctly as the LP) receives event messages from the second LP (which can also be referred to as the other LP). The steps described with reference to FIG. 9 can be performed by a controller (e.g., 112) of the first LP (e.g., 102a, 102b). The various values that are stored at the steps described with reference to FIG. 9 can be stored in the memory (e.g., 162) of the first LP that performs the steps.

Step 902 involves the first LP determining and storing a beginning low-resolution count value (B_low_res) of a low-resolution counter (e.g., 604 in FIG. 6) and a beginning high-resolution count value (B_high_res) of a high-resolution counter (e.g., 606 in FIG. 6) corresponding to when the first LP delivers pacing stimulation to the first cardiac chamber.

Step 902 involves the first LP monitoring for an event message from the second LP. Step 904 involves the first LP determining whether an event message is received from the second LP indicating that the second LP delivered (or is about to deliver) pacing stimulation to the second cardiac chamber in or on which the second LP is implanted. When the answer to step 904 is No, flow returns to step 904. When the answer to step 904 is Yes, flow goes to step 908.

Step 908 involves the first LP determining and storing a measured low-resolution count value (M_low_res) of the low-resolution counter (e.g., 604 in FIG. 6) and a measured high-resolution count value (M_high_res) of the high-resolution counter (e.g., 606 in FIG. 6) corresponding to when the first LP receives the event message, from the second LP, informing first LP that the second LP delivered (or is about to deliver) pacing stimulation to the second cardiac chamber.

Step 910 involves the first LP determining and storing a base rate low-resolution count value (BR_low_res) corresponding to how many counts of the low-resolution counter occur during a base pacing rate interval. The base rate pacing interval can be programmed by a physician or clinician or may have a default value. The base rate pacing interval can be, e.g., 1000 msec if the base rate is 60 beats per minute (bpm), 706 msec if the base rate is 85 bpm, or 750 msec if the base rate is 80 bpm, but is not limited thereto. An example range of values for the base rate pacing interval is from 600 msec to 1000 msec, but is not limited thereto. The first LP, and more specifically the controller (e.g., 112) thereof, can determine the base rate low-resolution count value (BR_low_res) by calculating how many low res-counts should occur during the base rate pacing interval. For example, if the base rate pacing interval is 1000 msec, and the frequency of the low resolution counter 604 is 128 Hz, then the base rate low-resolution count value (BR_low_res) would be equal to 128. For another example, if the base rate pacing interval is 750 msec, and the frequency of the low resolution counter 604 is 128 Hz, then the base rate low-resolution count value (BR_low_res) would be equal to 96. For another example, if the base rate pacing interval is 1000 msec, and the frequency of the low resolution counter 604 is 256 Hz, then the base rate low-resolution count value (BR_low res) would be equal to 256. For still another example, if the base rate pacing interval is 750 msec, and the frequency of the low resolution counter 604 is 256 Hz, then the base rate low-resolution count value (BR_low_res) would be equal to 192.

Step 912 involves the first LP determining and storing a remaining low-resolution count value (R_low_res) corresponding to the base rate low-resolution count value (BR_low_res) (determined at step 910) minus the measured low-resolution count value (M_low_res) (determined at step 908).

Referring again to step 910, while step 910 is shown in FIG. 9 as being performed between steps 908 and 910, step 910 can alternatively be performed at another time so long as the base rate low-resolution count value (BR_low_res) is determined and stored prior to step 912, such that it is available for use in the calculation performed at step 912. For example, step 910 can be performed prior to steps 902-910, and may even be performed prior to the first period of time during which the other count values described with reference to FIG. 9 are determined and stored.

Step 914 involves the first LP determining and storing an expected high-resolution count value (E_high_res) which is equal to the modulo n operation of a sum of the beginning high-resolution count value (B_high_res) (determined at step 902) and a calculated high-resolution count value (C_high_res). If the first LP is configured to be implanted in or on a ventricular chamber (e.g., the right ventricle) or proximate the LBB and to perform pacing of the ventricular chamber, then the calculated high-resolution count value (C_high_res) preferably corresponds to the base pacing rate interval minus a programmed AV interval. For example, if the base pacing rate interval is 1000 msec, the programmed AV interval is 200 msec, and the frequency of the high resolution counter is 32 kHz, then the calculated high-resolution count value (C_high_res) (which corresponds to the 1000 msec base pacing rate interval minus the 200 msec programmed AV interval, i.e., which corresponds to 800 msec) would equal 25,600. For another example, if the base pacing rate interval is 1000 msec, the programmed AV interval is 250 msec, and the frequency of the high resolution counter is 64 kHz, then the calculated high-resolution count value (C_high_res) (which corresponds to the 1000 msec base pacing rate interval minus the 250 msec programmed AV interval, i.e., which corresponds to 750 msec) would equal 48,000. Alternatively, if the first LP is configured to be implanted in or on an atrial chamber (e.g., the right atrium) and to perform pacing of the atrial chamber, then the calculated high-resolution count value (C_high_res) preferably corresponds to the programmed AV interval. For example, if the programmed AV interval is 200 msec, and the frequency of the high resolution counter is 32 kHz, then the calculated high-resolution count value (C_high_res) (which corresponds to the 200 msec programmed AV interval) would equal 6,400. An example range of values for the value "n" used in the modulo n operation is from 255 to 65535 (unsigned one byte to two bytes), but is not limited thereto. In a specific embodiment, the value for "n" used in the modulo n operation is 255.

Step 916, which provides for a specific implementation of step 806 introduced above in the discussion of FIG. 8, involves the first LP determining and storing as the compensation offset, the expected high-resolution count value (E_high_res) (determined at step 914) minus the measured high-resolution count value (M_high_res) (determined at step 908). In accordance with certain embodiments, steps 902-914 are repeated a plurality of times (e.g., 3 to 10 times) during the first period of time, during which the first LP (which can also be referred to more succinctly as the LP) receives event messages from the second LP (which can also be referred to as the other LP), and the first LP determines and stores the various count values described above. At step 916 the first LP can determine a plurality of compensation offsets based on the count value, and the first LP can determine an average compensation offset which is used during the second period of time as the compensation offset to compensate for drift between the timing circuitry of the first LP and the timing circuitry of the second LP so that the AV synchrony is maintained during the second period of time. In certain embodiments, a running average a plurality (e.g., 3 to 10) of most recently determined compensation offset values is determined and stored and is available for use as soon as the first LP stops receiving event messages from the second LP, at which time it would be beneficial to compensate for the aforementioned drift that may occur. Equivalently, the first LP can determine a respective average (e.g., a running average) of a plurality of each of the aforementioned count values it determines, and the compensation offset can be determined based on the averages of the aforementioned count values. In accordance with an embodiment, if both the first and the second LPs perform compensation (i.e., determine and utilize a compensation offset), then each of the first and the second LPs may store and utilize one-half of the compensation offset value determined in the above described manner.

Referring briefly back to FIG. 6, in accordance with certain embodiments, the high-resolution counter 606 is a modulo-n counter that is configured to count from zero to n-1 and then reset back to zero. In certain such embodiments, the expected high-resolution count value (E_high_res), which is used at step 914, is determined by performing a modulo-n operation on the sum of the beginning high-resolution count value (B_high_res) (determined at step 902) and the calculated high-resolution count value (C_high_res).

Referring now to FIG. 10, the steps shown in and described with reference to FIG. 10 can be considered sub-steps of step 808 in FIG. 8, in accordance with certain embodiments of the present technology. Such steps are performed during the second period of time, during which the first LP (which can also be referred to more succinctly as the LP) does not receive event messages from the second LP (which can also be referred to as the other LP). More generally, FIG. 10 is used to explain how the first LP times its pacing of the first cardiac chamber during the second period of time, based on the compensation offset that the first LP determined and stored at step 806 based on one or more count values determined during the first period of time at step 804. The steps described with reference to FIG. 10 can be performed by a controller (e.g., 112) of the first LP (e.g., 102a, 102b).

Step 1002 involves the first LP delivering pacing stimulation to the cardiac chamber in or on which the first LP is implanted. Step 1004 involves starting when the first LP most recently delivered pacing stimulation to the first cardiac chamber (at the most recent instance of step 1002), using the low-resolution counter to count to a calculated low-resolution count value (C_low_res) that is equal to the measured low-resolution count value (M_low_res) minus the beginning low-resolution count value (B_low_res). In a case where the beginning low-resolution count value (B_low_res) is equal to zero, the counting at step 1004 can simply be to the measured low-resolution count value (M_low_res). The beginning low-resolution count value (B_low_res) may have been determined at a most recent instance of step 902, or could be an average of a plurality of beginning low-resolution count value determined at a plurality of instances of step 902. The measured low-resolution count value (M_low_res) may have been determined at a most recent instance of step 908, or could be an average of a plurality of measured low-resolution count value determined at a plurality of instances of step 908. It would also be possible for a calculated low-resolution count value (C_low_res) to be determined each time steps 902 and 908 are performed, and the calculated low-resolution count value (C_low_res) used at steps 1002 and 1004 can be an average of a plurality of calculated low-resolution count values. Other variations are also possible and within the scope of the embodiments described herein. Explained another way, step 1004 involves initializing and starting the low-resolution counter (e.g., 604 in FIG. 6) to count to the calculated low-resolution count value (C_low_res). At step 1006 there is a determination of whether the low resolution counter has counted to the calculated low-resolution count value (C_low_res). When the answer to the determination at step 1006 is No, the flow returns to step 1006. When the answer to the determination at step 1006 is Yes, the flow goes to step 1008.

Step 1008 involves starting when the low-resolution counter finishes counting to the calculated low-resolution count value (C_low_res), using the high-resolution counter to count to the compensation offset. The compensation offset may have been determined at a most recent instance of step 916, or could be an average of a plurality of compensation offset values determined at a plurality of instances of step 916. Explained another way, step 1008 involves initializing and starting the high-resolution counter (e.g., 606 in FIG. 6) and using it to count to the compensation offset. At step 1010 there is a determination of whether the high-resolution counter has counted to the compensation offset. When the answer to the determination at step 1010 is No, the flow returns to step 1010. When the answer to the determination at step 1010 is Yes, the flow goes to step 1012.

Step 1012 involves starting when the high-resolution counter finishes counting to the compensation offset, using the low-resolution counter to count to the remaining low-resolution count value (R_low_res). The remaining low-resolution count value (R_low_res) may have been determined at a most recent instance of step 912, or could be an average of a plurality of remaining low-resolution count values determined at a plurality of instances of step 912. Explained another way, step 1012 involves initializing and starting the low-resolution counter (e.g., 604 in FIG. 6) and using it to count to the remaining low-resolution count value (R_low_res). At step 1014 there is a determination of whether the low resolution counter has counted to the remaining low-resolution count value (R_low_res). When the answer to the determination at step 1014 is No, the flow returns to step 1014. When the answer to the determination at step 1014 is Yes, flow returns to step 1002, and a further pacing stimulation is delivered to the first cardiac chamber. That is, when the low-resolution counter finishes counting to the low-resolution remaining count value (R_low_res), further pacing stimulation is delivered to the first cardiac chamber.

In an alternative embodiment, starting when the first LP most recently delivered pacing stimulation to the first cardiac chamber, the low-resolution counter 604 is used to count to the sum of the M_low_res + R_low_res, and then the high-resolution counter 606 is started and the further pacing stimulation is delivered to the first cardiac chamber when the high-resolution counter 606 finishes counting to the compensation offset.

In another alternative embodiment, starting when the first LP most recently delivered pacing stimulation to the first cardiac chamber, the high-resolution counter 606 is used to count to the compensation offset, and then the low-resolution counter 604 is started, and the further pacing stimulation is delivered to the first cardiac chamber when the low-resolution counter 604 finishes counting to the sum of the M_low_res + R_low_res.

More generally, following when the first LP most recently delivered pacing stimulation to the first cardiac chamber, the first LP delivers the further stimulation after the low resolution counter 604 has had a chance to count to C_low_res + R_low_res, and after the high-resolution counter 606 has had a chance to count to the compensation offset. The order of the counting doesn't matter so long as the further stimulation is delivered at a time (following a most recent pacing stimulation) equal to the sum of C_low_res + R_low_res + the compensation offset. Explained another way, starting when the first LP most recently delivered pacing stimulation to the first cardiac chamber, serially one after another in any order, the low-resolution counter 604 is used to count to the calculated low-resolution count value (C_low_res), the high-resolution counter 606 is used to count to the compensation offset, and the low-resolution counter 604 is used to count to the remaining low-resolution count value (R_low_res). In such an embodiment, using the low-resolution counter 604 to count to the calculated low-resolution count value (C_low_res) and to count to the remaining low-resolution count value (R_low_res) may be performed by using the low-resolution counter to count to a sum of the calculated low-resolution count value (C_low_res) and the remaining low-resolution count value (R_low_res). Further, in such an embodiment, the further pacing stimulation is delivered to the first cardiac chamber, when the low-resolution counter 604 has finished counting to the calculated low-resolution count value (C_low_res) and to the remaining low-resolution count value (R_low_res), or the sum thereof, and the high-resolution counter 606 has finished counting to the compensation offset, serially one after another in any order.

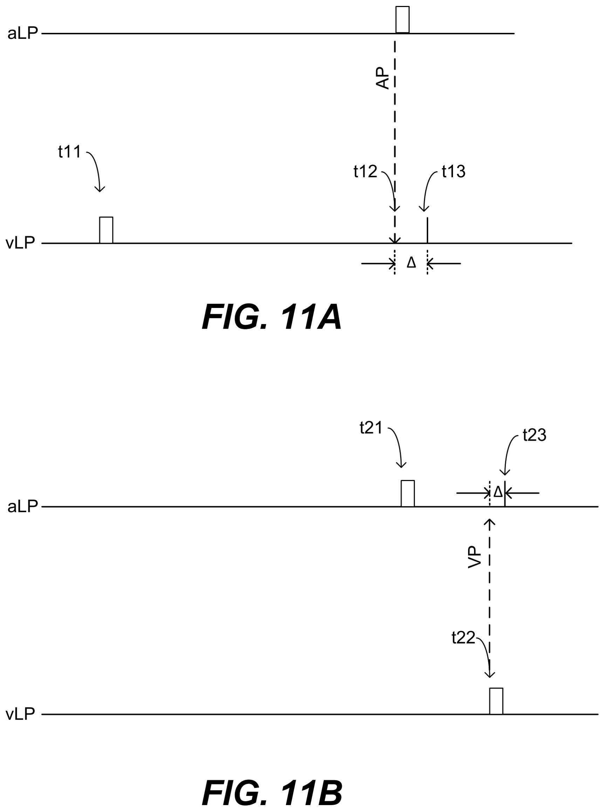

FIG. 11A illustrates a timing diagram that will now be used to illustrate the how the compensation offset, which is self-adaptive, can be determined in accordance with an embodiment of the present technology. More specifically, FIG. 11A illustrates how a vLP (e.g., 102b) that is configured to perform i2i communication with an aLP (e.g., 102a) can determine a compensation offset based on one or more count values determined during a first period of time that the vLP 102b receives event messages from the aLP 102a using i2i communication. At time t11, the vLP 102b determines and stores in its memory 162 a beginning low-resolution count value (B_low_res) of its low-resolution counter 604 and a beginning high-resolution count value (B_high_res) of its high-resolution counter 606 corresponding to when the vLP 102b delivers pacing stimulation to the ventricular cardiac chamber (e.g., the right ventricle) in or on which the vLP 102b is implanted. The vLP 102b thereafter monitors for an event message from the aLP 102a.

At time t12 the vLP 102b receives an event message from aLP 102a, informing vLP 102b that the aLP 102a delivered (or is about to deliver) pacing stimulation to the atrial chamber (e.g., the right atrium) in or on which the aLP 102a is implanted. Additionally, at time t12 the vLP 102b determines and stores in its memory 162 a measured low-resolution count value (M_low_res) of its low-resolution counter 604 and a measured high-resolution count value (M_high_res) of its high-resolution counter 606, corresponding to when the vLP 102b receives the event message, from the aLP 102a, informing vLP 102b that the aLP 102a delivered (or is about to deliver) pacing stimulation to the atrial cardiac chamber.

Thereafter, the vLP 102b determines and stores in its memory 162 a remaining low-resolution count value (R_low_res) corresponding to the base rate low-resolution count value (BR_low_res) (which can be predetermined, as discussed above) minus the measured low-resolution count value (M_low_res) determined and stored at time t12. Additionally, the vLP 102b determines and stores in its memory 162 an expected high-resolution count value (E_high_res) corresponding to a sum of the beginning high-resolution count value (B_high_res) (determined and stored at time t11) and a calculated high-resolution count value (C_high_res). In this case, since the vLP 102b is configured to be implanted in or on a ventricular chamber (e.g., the right ventricle) or proximate the LBB and to perform pacing of the ventricular chamber, the calculated high-resolution count value (C_high_res) corresponds to the base pacing rate interval minus a programmed AV interval.

Following time t12, the vLP 102b can determine and store in its memory 162 as the compensation offset, the expected high-resolution count value (E_high_res) (determined in the manner described above) minus the measured high-resolution count value (M_high_res) (determined and stored at time t12). For example, if the expected high-resolution count value (E_high_res) corresponded to time t13 in FIG. 11A, then the compensation offset would be a high-resolution count value corresponding to the delta between time t13 and time t12.

FIG. 11B illustrates how the aLP 102a can determine a compensation offset based on one or more count values determined during a first period of time that the aLP 102a receives event messages from the vLP 102b using i2i communication. At time t21, the aLP 102a determines and stores in its memory 162 a beginning low-resolution count value (B_low_res) of its low-resolution counter 604 and a beginning high-resolution count value (B_high_res) of its high-resolution counter 606 corresponding to when the aLP 102a delivers pacing stimulation to the atrial cardiac chamber (e.g., the right atrium) in or on which the aLP 102a is implanted. The aLP 102a thereafter monitors for an event message from the vLP 102b. At time t22 the aLP 102a receives an event message from vLP 102b, informing aLP 102a that the vLP 102b delivered (or is about to deliver) pacing stimulation to the ventricular chamber (e.g., the right ventricle) in or on which the vLP 102b is implanted. Additionally, at time t22 the aLP 102a determines and stores in its memory 162 a measured low-resolution count value (M_low_res) of its low-resolution counter 604 and a measured high-resolution count value (M_high_res) of its high-resolution counter 606, corresponding to when the aLP 102a receives the event message, from the vLP 102b, informing aLP 102a that the vLP 102b delivered (or is about to deliver) pacing stimulation to the ventricular cardiac chamber.

Thereafter, the aLP 102a determines and stores in its memory 162 a remaining low-resolution count value (R_low_res) corresponding to the base rate low-resolution count value (BR_low_res) (which can be predetermined, as discussed above) minus the measured low-resolution count value (M_low_res) determined and stored at time t22. Additionally, the aLP 102a determines and stores in its memory 162 an expected high-resolution count value (E_high_res) corresponding to a sum of the beginning high-resolution count value (B_high_res) (determined and stored at time t21) and a calculated high-resolution count value (C_high_res). In this case, since the aLP 102a is configured to be implanted in or on an atrial chamber (e.g., the right atrium) and to perform pacing of the atrial chamber, the calculated high-resolution count value (C_high_res) corresponds to the programmed AV interval.

Following time t22, the aLP 102a can determine and store in its memory 162 as the compensation offset, the expected high-resolution count value (E_high_res) (determined in the manner described above) minus the measured high-resolution count value (M_high_res) (determined and stored at time t22). For example, if the expected high-resolution count value (E_high_res) corresponded to time t23 in FIG. 11B, then the compensation offset would be a high-resolution count value corresponding to the delta between time t23 and time t22.

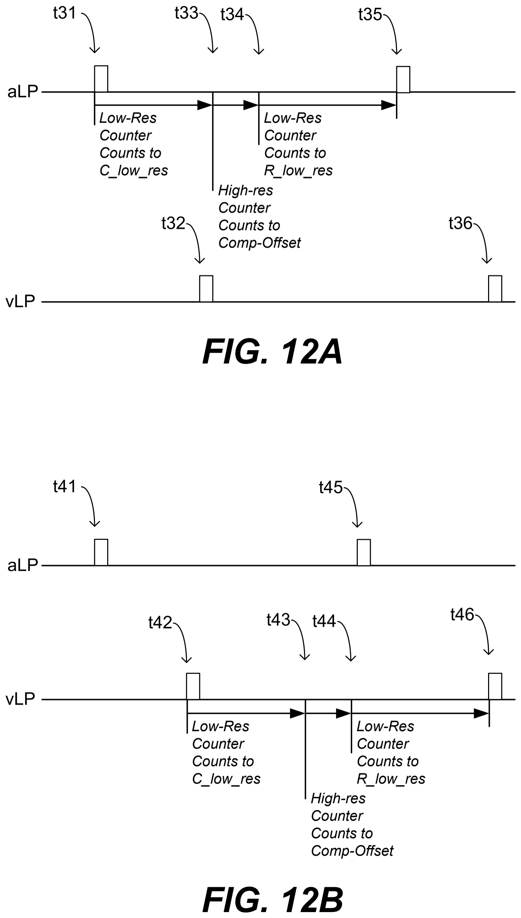

Reference is now made to FIG. 12A, which is a timing diagram illustrating operations of the aLP 102a and vLP 102b, during a period of time (e.g., one of the second periods of time described above) during which there is no i2i communications therebetween, and during which the aLP 102a uses an embodiment of the present technology to compensate for drift between timing circuitry of the aLP 102a and the vLP 102b. It is assumed that during a previous period of time (e.g., one of the first periods of time described above) during which there was i2i communication between the aLP 102a and the vLP, the aLP 102a determined and stored a measured low-resolution count value (M_low_res) at one or more instances of step 908, determined and stored a remaining low-resolution count value (R_low_res) at one or more instances of step 912, and determined and stored a compensation offset (aka delta) value at one or more instances of step 916. Where multiple instances of steps 908, 912, and 916 were performed, averages of the values determined during multiple instances of those steps could have been determined and stored and used to compensate for drift between timing circuitry of the aLP 102a and timing circuitry of the vLP 102b.

Referring to FIG. 12A, at time t31 a pacing stimulation is delivered by the aLP 102a to the right atrium in (or on) which the aLP 102a is implanted, in accordance with step 1002. Additionally, at time t31 a low-resolution counter (e.g., 604) of the aLP 102a is initialized and starts counting to the calculated low-resolution count value (C_low_res).

At time t33 the low-resolution counter (e.g., 604) of the aLP 102a finishes counting to the calculated low-resolution count value (C_low_res). Additionally at time t33 the high-resolution counter (e.g., 606) is initialized and starts counting to the compensation offset (aka delta) value, in accordance with step 1008. In an embodiment, initialization and starting of the high-resolution counter (e.g., 606) is triggered by the low-resolution counter (e.g., 604) finishing its counting to the calculated low-resolution count value (C_low_res).

At time t34 the high-resolution counter (e.g., 606) finishes counting to the compensation offset (aka delta) value. Additionally at time t34 the low-resolution counter (e.g., 604) is initialized and starts counting to the remaining low-resolution count value (R_low_res), in accordance with step 1012. In an embodiment, reinitialization and restarting of the low-resolution counter (e.g., 606) is triggered by the high-resolution counter (e.g., 606) finishing its counting to the compensation offset (aka delta) value.

At time t35 the low-resolution counter (e.g., 604) of the aLP 102a finishes counting to the remainder low-resolution count value (R_low_res) and a further pacing stimulation is delivered by the aLP 102a to the right atrium in (or on) which the aLP 102a is implanted, in accordance with a further instance of step 1002. In an embodiment, the delivery of the further pacing stimulation is triggered by the low-resolution counter (e.g., 604) of the aLP 102a finishes counting to the remainder low-resolution count value (R_low_res). The aLP 102a delivering further pacing stimulation at time t35 to the atrial chamber (in or on which the aLP 102a is implanted) is an example of the aLP 102a delivering further pacing stimulation at a compensated VA interval following an expected time at which the vLP 102b delivers pacing stimulation (to the ventricular chamber in or on which the vLP 102b is implanted).

FIG. 12A also illustrates that at a time t32 a pacing stimulation is delivered by the vLP 102b to the right ventricle in (or on) which the vLP 102b is implanted. Additionally, FIG. 12B illustrates that at a time t36 a further pacing stimulation is delivered by the vLP 102b to the right ventricle in (or on) which the vLP 102b is implanted.

In certain embodiments, so long as the aLP 102a is using an embodiment of the present technology to compensate for drift between the aLP 102a and the vLP 102b, the vLP 102b does not attempt to compensate for the drift. In such a case, the vLP 102b can deliver its ventricular pacing stimulation in accordance with its base pacing rate or base pacing rate interval. It would also be possible for the pacing rate and/or pacing rate interval to vary responsive to temperature sensed by a temperature sensor (e.g., 152) and/or activity sensed by an accelerometer (e.g., 154), in which case the DOO operation can be rate responsive, i.e., DOOR. More generally, each previous mention of a base pacing rate and/or base pacing rate interval can be adjusted responsive to temperature sensed by a temperature sensor (e.g., 152) and/or activity sensed by an accelerometer (e.g., 154).

Reference is now made to FIG. 12B, which is a timing diagram illustrating operations of the aLP 102a and vLP 102b, during a period of time (e.g., one of the second periods of time described above) during which there is no i2i communications therebetween, and during which the vLP 102b uses an embodiment of the present technology to compensate for drift between respective timing circuitry 160 of the vLP 102b and the aLP 102a. It is assumed that during a previous period of time (e.g., one of the first periods of time described above) during which there was i2i communication between the aLP 102a and the vLP 102b, the vLP 102b determined and stored a measured low-resolution count value (M_low_res) at one or more instances of step 908, determined and stored a remaining low-resolution count value (R_low_res) at one or more instances of step 912, and determined and stored a compensation offset (aka delta) value at one or more instances of step 916. Where multiple instances of steps 908, 912, and 916 were performed, averages of the values determined during multiple instances of those steps could have been determined and stored and used to compensate for drift between respective timing circuitry 160 of the vLP 102b and the aLP 102a. Additionally, the vLP 102b can be determine and store a calculated low-resolution count value (C_low_res) that is equal to the measured low-resolution count value (M_low_res) minus the beginning low-resolution count value (B_low_res).

Referring to FIG. 12B, at time t42 a pacing stimulation is delivered by the vLP 102b to the right ventricle in (or on) which the vLP 102b is implanted, in accordance with step 1002. Additionally, at time t42 a low-resolution counter (e.g., 604) of the vLP 102b is initialized and starts counting to the calculated low-resolution count value (C_low_res).

At time t43 the low-resolution counter (e.g., 604) of the vLP 102b finishes counting to the calculated low-resolution count value (C_low_res). Additionally at time t43 the high-resolution counter (e.g., 606) is initialized and starts counting to the compensation offset (aka delta) value, in accordance with step 1008. In an embodiment, initialization and starting of the high-resolution counter (e.g., 606) is triggered by the low-resolution counter (e.g., 604) finishing its counting to the calculated low-resolution count value (C_low_res).

At time t44 the high-resolution counter (e.g., 606) finishes counting to the compensation offset (aka delta) value. Additionally at time t44 the low-resolution counter (e.g., 604) is initialized and starts counting to the remaining low-resolution count value (R_low_res), in accordance with step 1012. In an embodiment, reinitialization and restarting of the low-resolution counter (e.g., 606) is triggered by the high-resolution counter (e.g., 606) finishing its counting to the compensation offset (aka delta) value.

At time t46 the low-resolution counter (e.g., 604) of the vLP 102b finishes counting to the remainder low-resolution count value (R_low_res) and a further pacing stimulation is delivered by the vLP 102b to the right atrium, in (or on) which the aLP 102a is implanted, in accordance with a further instance of step 1002. In an embodiment, the delivery of the further pacing stimulation is triggered by the low-resolution counter (e.g., 604) of the vLP 102b finishes counting to the remainder low-resolution count value (R_low_res). The vLP 102b delivering further pacing stimulation at time t46 to the ventricular chamber (in or on which the vLP 102b is implanted) is an example of the vLP 102b delivering further pacing stimulation at a compensated AV interval following an expected time at which the aLP 102a delivers pacing stimulation (to the atrial chamber in or on which the aLP 102a is implanted).

FIG. 12B also illustrates that at a time t41 a pacing stimulation is delivered by the aLP 102a to the right atrium in (or on) which the aLP 102a is implanted. Additionally, FIG. 12B illustrates that at a time t45 a further pacing stimulation is delivered by the aLP 102a to the right atrium in (or on) which the aLP 102a is implanted.

In certain embodiments, so long as the vLP 102b is using an embodiment of the present technology to compensate for drift between the vLP 102b and the aLP 102a, the aLP 102a does not attempt to compensate for the drift. In such a case, the aLP 102a can deliver its atrial pacing stimulation in accordance with its base pacing rate or base pacing rate interval. It would also be possible for the pacing rate and/or pacing rate interval to vary responsive to temperature sensed by a temperature sensor (e.g., 152) and/or activity sensed by an accelerometer (e.g., 154), in which case the DOO operation can be rate responsive, i.e., DOOR. More generally, each previous mention of a base pacing rate and/or base pacing rate interval can be adjusted responsive to temperature sensed by a temperature sensor (e.g., 152) and/or activity sensed by an accelerometer (e.g., 154).

In certain embodiments, each the aLP 102a and the vLP 102b independently implement an embodiment of the present technology to compensate for drift between the aLP 102a and the vLP 102b. For example, each of the aLP 102a and the vLP 102b can perform the steps described above with reference to FIG. 8. Further, each of the aLP 102a and the vLP 102b can perform the steps described above with reference to FIG. 9 and determine and store the values described therein, and use those values to perform the steps described with reference to FIG. 10.

Each of the aforementioned counters can be count-up or count-down timers. Where a counter is a count-up timer, it can be initialized to zero and then count up to a specified value, in response to which another counter and/or action can be triggered. Where a counter is a count-down timer, it can be initialized to a specified count value and then count down to zero, in response to which another counter and/or action can be triggered. Either way, the counters can be used to count a specified number of counts corresponding to a specified count value.

In embodiments where the first LP is an aLP (e.g., 102a) and the second LP is a vLP (e.g., 102b), or where the first LP is a vLP (e.g., 102b) and the second LP is an aLP (e.g., 102a), the embodiments described above with reference to FIGS. 6-12 enable the first LP and the second LP to collectively operate in the DOO mode and compensate for drift during periods of time that the first LP does not receive event messages from the second LP. The DOO mode, as explained above, provides for dual chamber pacing, i.e., pacing in both atrial pacing (that is performed by the aLP) and ventricular pacing (that is performed by the vLP). Accordingly, a benefit of the embodiments described herein is that a dual chamber LP system can continue to operate in a DOO mode in the presence of environmental noise, e.g., due to a patient being exposed to or in close proximity to a magnetic resonance imaging (MRI) machine or being exposed to other environmental nose. A further benefit of the embodiments described herein is that a dual chamber LP system can continue to operate in a DOO mode if one or both of the LPs temporarily abstain from sending event messages to the other LP in order to conserve power, or if one of the LP disables at least a portion of its receiver to conserve energy, and/or if a communication channel between the first and second LPs becomes unstable.

Another benefit of the embodiments described herein is that drift compensation can be performed after the first LP and the second LP are implanted. Additionally, with the embodiments described herein there is no need for a predefined pair of LPs to have their timing (e.g., oscillators, clocks and/or counters) trimmed relative to one another prior to implantation and/or after implantation of the pair of LPs. Also, with the embodiments described herein, if the drift changes over time due to changes to the battery of one or both of the LPs, changes to temperature, and/or other environmental changes, the compensation offset that is determined and used to compensate for drift will automatically be appropriately updated to compensation for the changes in the drift over time.

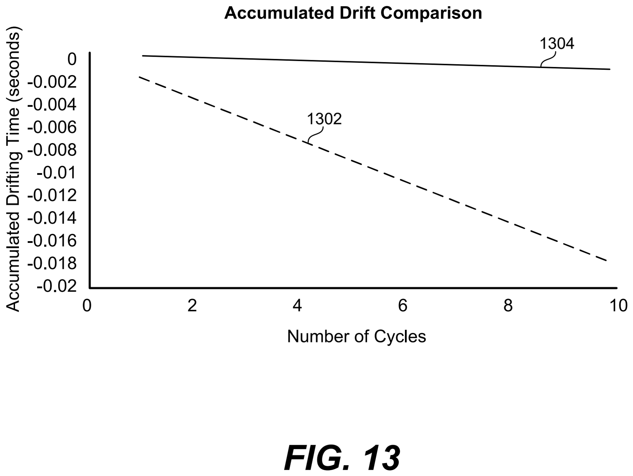

Collected pace interval data was used to perform a drift analysis to compare the drift that occurs without using an embodiment of the present technology to the drift that would occur using an embodiment of the present technology. A drift compensation model was determined by taking the first 10 pacing cycles as the last sliding window before entering an EMI interruption session. The determined compensation model was applied to the next 10 pacing cycle and the accumulated drift improvement (with vs. without drift compensation) is shown in the graph of FIG. 13. More specifically, the hatched line 1302 in FIG. 13 illustrates drift that occurs between a first LP and a second LP over 10 cardiac cycles during which the first LP does not receive event messages from the second LP, wherein the drift is not compensated for using an embodiment of the present technology. The solid line 1304 in FIG. 13 illustrates drift that occurs between a first LP and a second LP over 10 cardiac cycles during which the first LP does not receive event messages from the second LP, wherein the drift is compensated for using an embodiment of the present technology described herein. As can be appreciated from FIG. 13, the cumulative drift over 10 cardiac cycles was shown as being reduced from about 0.018 seconds (i.e., 18 msec) to less than 0.001 seconds (i.e., less than 1 msec) by using an embodiment of the present technology.

At noted above, it is possible that the aLP 102a and the vLP 102b can be configured to operate at least some of the time in an AAI+VVI mode (which can also be referred to as AAI+VVI operation) during which the aLP and the vLP purposely abstain from communicating with one another using i2i communication to conserve their energy and thereby increase their longevity. Additional details of the AAI+VVI operation that may be performed by the aLP 102a and the vLP 102b are described in U.S. Patent Application No. 18/819,947, titled "DUAL CHAMBER LEADLESS PACEMAKER SYSTEMS AND METHODS FOR USE THEREWITH," filed on August 29, 2024, which published as US20250108221 A1 on April 3, 2025.

In accordance with certain embodiments of the present technology, the aLP 102a and the vLP 102b can, during a first period of time, transmit event messages to one another to thereby enable the aLP 102a and the vLP 102b to collectively provide a coordinated dual chamber operation, such as DOO operation. Additionally, during the first period of time the aLP 102a and/or the vLP 102b determines and stores one or more of the count values discussed above, which are briefly discussed again below. Thereafter, during a second period of time, the aLP 102a and the vLP 102b can purposely abstain from transmitting event messages to one another to thereby conserve power. In certain embodiments, during this second period of time, during which the aLP 102a and the vLP 102b purposely abstain from transmitting event messages to one another to thereby conserve power, the aLP 102a and the vLP 102b operate in the AAI+VVI mode, during which the aLP 102a provides AAI operation and the vLP 102b provides VVI operation.

As noted above, the AAI operation and the VVI operation do not depend on event messages being transmitted between the aLP 102a and the vLP 102b. However, whenever the vLP 102b delivers ventricular pacing during two or more consecutive cardiac cycles while operating in the VVI mode, if there is any drift between the timing circuitry 160 of the aLP 102a and the timing circuitry 160 of the vLP 102b, it is possible that the vLP may provide for less than optimal synchronization between atrial and ventricular pacing. In certain embodiments, during at least one of the first period of time or the second period of time, at least one of the aLP 102a or the vLP 102b is configured to determine a compensation offset based on at least one of the one or more count values determined and stored during the first period of time. The compensation offset, as noted above, is indicative of a drift between the timing circuitry 160 of the aLP 102a and the timing circuitry 160 of the vLP 102b. This way, during the second period of time, during which the aLP 102a and the vLP 102b purposely abstain from transmitting event messages to one another to conserve power, and during with AAI+VVI operation is performed, at least one of the aLP 102a or the vLP 102b can use the compensation offset to compensate for the drift between the timing circuitry160 of the aLP 102a and the timing circuitry 160 of the vLP 102b so that synchronization between atrial and ventricular pacing can be maintained during the second period of time whenever vLP provides ventricular pacing for two or more consecutive cardiac cycles while providing the VVI operation.

In certain embodiments, it is the vLP 102b that stores the one or more count values, determines the compensation offset based thereon, and then uses the compensation offset during the second period of time (during which the aLP 102a and the vLP 102b abstain from transmitting event messages to one another to thereby conserve power, the aLP 102a is provides the AAI operation, and the vLP 102b is provides the VVI operation). More specifically, it may be more practical for the vLP 102b to determine and use the compensation offset since the vLP 102b will most likely deliver ventricular pacing less often while performing the VVI operation than the aLP 102a delivers atrial pacing while performing the AAI operation. This is because a primary use case for utilizing the AAI+VVI mode is with patients having sinus node dysfunction (SND), where atrial-based pacing is the primary therapeutic support needed and the patient generally has intact AV node conduction, with potentially a rare-to-occasional need for intermittent ventricular pacing support, e.g., due to transient AV block, but not limited thereto. In this context, when a dual chamber LP system is utilizing the AAI+VVI operating mode, the primary role of the vLP 102b is to provide backup ventricular safety pacing support, which optimally minimizes ventricular pacing to be only when needed by the patient and when pacemaker-mediated atrioventricular (AV) synchrony is not a clinical necessity. That is, the vLP 102b is generally used to provide backup ventricular safety pacing support when the AAI+VVI operating mode is being provided. Nevertheless, while the vLP 102b is providing the VVI operation, there can be periods of time during which the vLP 102b will need to deliver ventricular pacing during two or more consecutive cardiac cycles. The vLP 102b can improve the synchronization between atrial and ventricular pacing during such periods of time (during which the vLP 102b delivers ventricular pacing during two or more consecutive cardiac cycles) by using the compensation offset to compensate for drift between the timing circuitry of the aLP 102a and the time circuitry of the vLP 102b.

During the AAI+VVI operation, when the aLP 102a provides the AAI operation, the aLP 102a performs atrial pacing when an intrinsic atrial event is not detected within a specified AA interval following a previous paced or intrinsic atrial event, performs atrial sensing, and inhibits the atrial pacing when the intrinsic atrial event is detected within the specified AA interval following the previous paced or intrinsic atrial event. Additionally, during the AAI+VVI operation, when the vLP 102b provides the VVI operation, the vLP 102b performs ventricular pacing when an intrinsic ventricular event is not detected within a specified VV interval following a previous paced or intrinsic ventricular event, performs ventricular sensing, and inhibits the ventricular pacing when the intrinsic ventricular event is detected within the specified VV interval following the previous paced or intrinsic ventricular event.

In certain embodiments, during the first period of time, during which the aLP 102a and the vLP 102b transmit event messages to one another, the vLP 102b determines and stores a measured count value corresponding to a duration between when the aLP 102a delivers pacing stimulation to the atrial chamber and when the aLP 102a receives an event message from the vLP 102b, informing aLP 102a that the vLP 102b delivered or is about to deliver pacing stimulation to the ventricular chamber. Additionally, the vLP 102b determines the compensation offset by subtracting the measured count value from an expected count value, wherein the expected count value corresponds to what the measured count value would be if there was no drift between the timing circuitry 160 of the aLP 102a and the timing circuitry 160 of the vLP 102b.

More specifically, as can be appreciated from the above discussion, assuming it is the vLP 102b that determines the compensation offset (and thereafter uses the compensation offset whenever the vLP 102b delivered pacing stimulation to the ventricular chamber during an immediately preceding cardiac cycle while providing the VVI operation), it will be the vLP 102b that determines and stores one or more count values during the first period of time, during which the aLP 102a and the vLP 102b are collectively providing DOO operation. More specifically, during the first period of time the vLP 102b may determine and store the following count values:
- a beginning low-resolution count value (B_low_res) of a low-resolution counter 604 and a beginning high-resolution count value (B_high_res) of a high-resolution counter 606 corresponding to when the vLP 102b delivers pacing stimulation to ventricular chamber;
- a measured low-resolution count value (M_low_res) of the low-resolution counter 604 and a measured high-resolution count value (M_high_res) of the high-resolution counter 606 corresponding to when the vLP 102b receives one of the event messages, from the aLP 102a, informing the vLP 102b that the aLP 102a delivered or is about to deliver pacing stimulation to the atrial chamber;
- a remaining low-resolution count value (R_low_res) corresponding to a base rate low-resolution count value (BR_low_res) minus the measured low-resolution count value (M_low_res); and
- an expected high-resolution count value (E_high_res) determined based on a sum of the beginning high-resolution count value (B_high_res) and a calculated high-resolution count value (C_high_res).

The base rate low-resolution count value (BR_low_res) may correspond to how many counts of the low-resolution counter 604 occur during a base pacing rate interval. For the vLP 102b, the base pacing rate interval is the VV interval. Accordingly, where it is the vLP 102b that determines the compensation offset, the base rate low-resolution count value (BR_low_res) may correspond to how many counts of the low-resolution counter 604 occur during a VV interval. The calculated high-resolution count value (C_high_res) may correspond to the base pacing rate interval minus a programmed AV interval. In certain embodiments, the vLP 102b determines as the compensation offset a result of the expected high-resolution count value (E_high_res) minus the measured high-resolution count value (M_high_res).

It is beneficial that the vLP 102b uses the compensation offset whenever the vLP 102b needs to deliver pacing stimulation to the ventricular chamber and had delivered pacing stimulation to the ventricular chamber during an immediately preceding cardiac cycle, and thus, whenever the vLP 102b is to deliver ventricular pacing during two or more consecutive cardiac cycles to thereby enable synchronization between atrial and ventricular pacing to be maintained. Details of how the vLP 102b can time its pacing of the ventricular chamber during the second period of time (during which the aLP 102a and the vLP 102b abstain from transmitting event messages to one another) based on the compensation offset that the vLP 102b determines and stores were described above in great detail, and thus, need not be discussed again. Nevertheless, for the convenience of the reader certain details are explained again below.

In certain embodiments, starting when the vLP 102b most recently delivered pacing stimulation to the ventricular chamber (in or on which the vLP 102b is implanted) during an immediately preceding cardiac cycle, serially one after another in any order, the vLP 102b uses its low-resolution counter 604 to count to a calculated low-resolution count value (C_low_res), and uses its high-resolution counter 606 to count to the compensation offset, and uses its low-resolution counter 604 to count to the remaining low-resolution count value (R_low_res). As was explained above, the calculated low-resolution count value (C_low_res) may be equal to the measured low-resolution count value (M_low_res) minus the beginning low-resolution count value (B_low_res). Additionally, as was explained above, use of the low-resolution counter 604 to count to the calculated low-resolution count value (C_low_res) and to count to the remaining low-resolution count value (R_low_res) may be performed by using the low-resolution counter 604 to count to a sum of the calculated low-resolution count value (C_low_res) and the remaining low-resolution count value (R_low_res). In such an embodiment, the vLP 102 delivers further pacing stimulation to the ventricular chamber, when the low-resolution counter 604 has finished counting to the calculated low-resolution count value (C_low_res) and to the remaining low-resolution count value (R_low_res), or the sum thereof, and the high-resolution counter 606 has finished counting to the compensation offset, serially one after another in any order, if the vLP 102b has not detected an intrinsic ventricular event prior to finishing all of the aforementioned counting. If during a cardiac cycle the vLP 102b detects an intrinsic ventricular event during its above described counting, then the vLP 102b will not deliver ventricular pacing during that cardiac cycle, and optionally can stop the aforementioned counting for that cardiac cycle.

While the aLP 102a and the vLP 102b are operating in the AAI+VVI operation, and while at least one of the aLP 102a and the vLP 102b uses the compensation offset to compensate for the drift between the timing circuitry160 of the aLP 102a and the timing circuitry 160 of the vLP 102b, the vLP 102b and/or the aLP 102a may monitor for certain criterion to determine whether the LPs should mode switch from the AAI+VVI operation back to DOO operation. For example, as was described in U.S. Patent Application No. 18/819,947, which was mentioned above, the vLP 102b can monitor for a first specified criterion, which if satisfied, would indicate that there should be a mode switch from the AAI+VVI operation to a coordinated dual chamber operation such as DOO operation. In accordance with certain embodiments, the first specified criterion, which the vLP 102b determines whether or not is satisfied, is the vLP 102b having provided at least a first specified threshold amount of ventricular pacing within a first specified duration. For example, the first specified criterion could be whether the vLP 102b delivered ventricular pacing pulses during at least a specified percent (e.g., 70 percent, or some other percent) of most recent cardiac cycles, or during at least a specified number N (e.g., N = 7, or some other number) of cardiac cycles during the most recent number M (e.g., M = 10, or some other number > N) of cardiac cycles, or over some specified duration of time (e.g., 1 minute, 2 minutes, or 5 minutes, etc.). Other variations are also possible and within the scope of the embodiments described herein. The vLP 102b abstains from using its transmitter to transmit any i2i messages to the aLP 102a (or to a third IMD that acts as a communication hub for the LPs) that are intended to be used by the aLP 102a to coordinate pacing with the vLP 102b while the vLP 102b is providing the VVI operation. While the vLP 102b abstains from using its transmitter to transmit such i2i messages to the aLP 102a, the vLP 102b may also disable its receiver, or at least a portion thereof, to further conserver power.

So long as the vLP 102b continues to provide the VVI operation, the aLP 102a can continue to provide the AAI operation. When the vLP 102b determines that first specified criterion is satisfied, the vLP 102b can transmit a mode switch type i2i message to the aLP 102a. The vLP 102b may enable its transmitter thereof (if the transmitter had been disabled to conserve power) to transmit i2i messages to the aLP 102a, and the vLP 102b may also enable one or more of its receiver(s) (if the receiver(s) thereof had been previously disabled to conserve power) to listen for a mode switch ACK i2i message that may be transmitted by the aLP 102a. More generally, the vLP 102b may determine that the dual chamber LP system should switch from AAI+VVI operation to coordinated dual chamber operation such as DOO operation when a patient's need for ventricular pace support becomes more than just rare or intermittent.

After the aLP 102a and the vLP 102b have mode switched to the dual chamber pacing mode, such as DOO operation, new count values can be stored to enable at least one of the aLP 102a or the vLP 102b to determine a new compensation offset that can be used the next time the LPs switch back to operating in the AAI+VVI mode. Further, while operating in the dual chamber pacing mode, e.g., DOO operation, the vLP 102b may monitor for a second specified criterion, which if satisfied, would indicate that there should be a mode switch from the coordinated dual chamber operation back to the AAI+VVI operation. In accordance with certain embodiments, the second specified criterion, which the vLP 102b determines whether or not is satisfied, is the vLP 102b having provided less than a second specified threshold amount of ventricular pacing within a second specified duration. For example, the second specified criterion could be whether the vLP 102b delivered ventricular pacing pulses during less than a specified percent (e.g., 30 percent, or some other percent) of most recent cardiac cycles, or during less than a specified number N (e.g., N = 30, or some other number) of cardiac cycles during the most recent number M (e.g., M = 100, or some other number > N) of cardiac cycles, or over some specified duration of time (e.g., 1 minute, 2 minutes, or 5 minutes, etc.). When the vLP 102b determines that the second specified criterion was satisfied, the vLP 102b can transmit a further mode switch type i2i message to the aLP 102a (or to the third IMD that acts as a communication hub for the LPs). More generally, there can be a determination of whether the dual chamber LP system 100 should switch from the coordinated dual chamber operation back to the AAI+VVI because the patient's need for ventricular pacing returned to be relatively rare or intermittent.

In accordance with certain embodiments, if the AAI operation performed by the aLP 102a is rate responsive (i.e., is more specifically AAIR operation) while the aLP 102a and the vLP 102b are collectively operating in the AAI+VVI operation, whenever the aLP 102a changes its AA interval (or more generally, its base pacing rate) due to a change in an activity level of the patient (as detected using an accelerometer 154 and/or a temperature sensor 152 of the aLP 102a), the aLP 102a sends an i2i message to the vLP 102b to inform the vLP 102b of the change in its base pacing rate to thereby enable the vLP 102b to appropriately adjust its VV interval (or more generally, its base pacing rate). Alternatively, the vLP 102b can independently also detect changes in the activity level of the patient (using an accelerometer 154 and/or a temperature sensor 152 of the vLP 102b) and can adjust its VV interval (or more generally, its base pacing rate) independently of the aLP 102a adjusting its AA interval (or more generally, its base pacing rate).

In accordance with certain embodiments, during AAI-VVI operation the pacing rate for the aLP 102a is restricted to always being greater than the pacing rate for the vLP 102b to ensure that the aLP 102a is driving overall cardiac electromechanical functionality, and therein mitigates against the vLP rate superseding the aLP rate, which can result in undesirable retrograde AV node conduction. This constraint may be achieved by programming the AA interval (that is used by the aLP 102a) to be shorter than the VV interval (that is used by the vLP 102b), which has the effect of causing an atrial rate of the atrial pacing performed by the aLP 102a to be faster than a ventricular rate of the ventricular pacing performed by the vLP 102b. Additionally or alternatively, in accordance with certain embodiments, the programmable pacing rate for the vLP 102b is restricted to always being less than the programmable pacing rate for the aLP 102a to ensure that the aLP is driving overall cardiac electromechanical functionality, and therein mitigates against the vLP rate superseding the aLP rate, which can result in undesirable retrograde AV node conduction. This constraint may be achieved by programming the VV interval (that is used by the vLP) to be longer than the AA interval (that is used by the aLP), which has the effect of causing a ventricular rate of the ventricular pacing performed by the vLP 102b to be slower than an atrial rate of the atrial pacing performed by the aLP 102a. In certain embodiments the aLP 102a may provide for rate responsive pacing, i.e., the AAI operation is provided by AAIR operation, while the vLP provides for non-rate responsive VVI pacing. More specifically, the aLP 102a may utilize a temperature sensor (e.g., 152) thereof and/or a motion sensor (e.g., accelerometer 154) thereof to detect patient activity, and modulate its pacing rate based on the detected patient activity. In certain such embodiments, while the aLP is providing rate responsive AAI operation, i.e., AAIR operation, the vLP provides its VVI operation utilizing a constant programmed ventricular rate. In other words, the VVI operation provided by the vLP is not rate responsive. This can be because the vLP disables its temperature sensor (e.g., 152) and/or its motion sensor (e.g., accelerometer 154), ignores the outputs thereof, or turns off its rate responsive feature. In other embodiments, while the aLP 102a is providing rate responsive AAI operation, i.e., AAIR operation, the vLP 102b is providing rate responsive VVI operation, i.e., VVIR operation, which can be referred to more specifically as AAIR+VVIR operation. In certain embodiments, when the dual chamber LP system 100 is providing AAIR+VVIR operation, a sensitivity of the temperature sensor and/or motion sensor of the vLP 102b is less than a sensitivity of the temperature sensor and/or motion sensor of the aLP 102a, so that an increase in the atrial pacing rate provided by aLP 102a is greater than a corresponding increase in the ventricular pacing rate provided by vLP 102b, which keeps the AA interval shorter than the VV interval to thereby cause an atrial rate of the atrial pacing performed by the aLP to remain faster than a ventricular rate of the ventricular pacing performed by the vLP.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are example embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A leadless pacemaker, LP, (102a, 102b) configured to communicate with a second LP (102b, 102a) using implant-to-implant, i2i, communication, wherein the LP (102a, 102b) is configured to perform pacing of a first cardiac chamber, and wherein the second LP (102b, 102a) is configured to perform pacing of a second cardiac chamber, the LP (102a, 102b) comprising:
a receiver (120, 122) configured to receive event messages from the second LP (102b, 102a) using i2i communication;
a plurality of electrodes (108a, 108b);
a memory (162);
a timing circuitry (160);
a pulse generator (116) configured to deliver pacing pulses using at least two of the plurality of electrodes (108a, 108b); and
a controller (112) communicatively coupled to the receiver (120, 122), the pulse generator (116), and the memory (162);
the controller (112) configured to:
during a first period of time, during which event messages are received by the receiver (120, 122) from the second LP (102b, 102a) using i2i communication, control the pulse generator (116) to time pacing of the first cardiac chamber based on the event messages received by the receiver (120, 122) from the second LP (102b, 102a) using i2i communication so that atrioventricular, AV, synchrony is maintained during the first period of time;
during the first period of time, during which the event messages are received by the receiver (120, 122) from the second LP (102b, 102a) using i2i communication, determine and store, in the memory (162), one or more count values produced using one or more timers of the timing circuitry (160) of the LP (102a, 102b);
determine a compensation offset based on at least one of the one or more count values determined and stored during the first period of time, wherein the compensation offset is indicative of a drift between the timing circuitry (160) of the LP (102a, 102b) and timing circuitry (160) of the second LP (102b, 102a); and
during a second period of time, during which event messages are not received by the receiver (120, 122) from the second LP (102b, 102a), control the pulse generator (116) to time pacing of the first cardiac chamber based on the compensation offset, to thereby compensate for the drift between the timing circuitry (160) of the LP (102a, 102b) and the timing circuitry (160) of the second LP (102b, 102a) so that the AV synchrony is maintained during the second period of time.

2. The LP (102a, 102b) of claim 1, wherein the controller (112) is configured to:
determine and store, in the memory (162) and during the first period of time, a measured count value corresponding to a duration between when the controller (112) controls the pulse generator (116) to deliver pacing stimulation to the first cardiac chamber and when the receiver (120, 122) receives an event message, from the second LP (102b, 102a), informing the LP (102a, 102b) that the second LP (102b, 102a) delivered or is about to deliver pacing stimulation to the second cardiac chamber; and
determine the compensation offset by subtracting the measured count value from an expected count value, wherein the expected count value corresponds to what the measured count value would be if there was no drift between the timing circuitry (160) of the LP (102a, 102b) and the timing circuitry (160) of the second LP (102b, 102a).

3. The LP (102a, 102b) of any one of claims 1 or 2, wherein:
the timing circuitry (160) of the LP (102a, 102b) comprises a high-resolution counter (606) and a low-resolution counter (604);
the controller (112) is configured to use the high-resolution counter (606) to determine, during the first period of time, and store in the memory (162), an expected high-resolution count value, E_high_res, and a measured high-resolution count value, M_high_res; and
the controller (112) is configured to determine as the compensation offset the expected high-resolution count value, E_high_res, minus the measured high-resolution count value, M_high_res.

4. The LP (102a, 102b) of claim 3, wherein:
the controller (112) is configured to use the low-resolution counter (604) to determine, during the first period of time, and store in the memory (162), a calculated low-resolution count value, C_low_res, and a remaining low-resolution count value, R_low_res; and
the controller (112) is configured to control the pulse generator (116), during the second period of time, to deliver further pacing stimulation to the first cardiac chamber, when the low-resolution counter (604) has finished counting to the calculated low-resolution count value, C_low_res, and to the remaining low-resolution count value, R_low_res, or the sum thereof, and the high-resolution counter (606) has finished counting to the compensation offset, serially one after another in any order.

5. The LP (102a, 102b) of any one of claims 1 through 4, wherein the timing circuitry (160) of the LP (102a, 102b) comprises:
a low-resolution counter (604); and
a high-resolution counter (606);
wherein during the first period of time, the controller (112) is configured to determine and store, in the memory (162), each of the following:
a beginning low-resolution count value, B_low_res, of the low-resolution counter (606) and a beginning high-resolution count value, B_high_res, of the high-resolution counter (606) corresponding to when the controller (112) controls the pulse generator (116) to deliver pacing stimulation to the first cardiac chamber;
a measured low-resolution count value, M_low_res, of the low-resolution counter (606) and a measured high-resolution count value, M_high_res, of the high-resolution counter (606) corresponding to when the receiver (120, 122) receives one of the event messages, from the second LP (102b, 102a), informing the LP (102a, 102b) that the second LP (102b, 102a) delivered or is about to deliver pacing stimulation to the second cardiac chamber;
a remaining low-resolution count value, R_low_res, corresponding to a base rate low-resolution count value, BR_low_res, minus the measured low-resolution count value, M_low_res; and
an expected high-resolution count value, E_high_res, determined based on a sum of the beginning high-resolution count value, B_high_res, and a calculated high-resolution count value, C_high_res;
wherein the base rate low-resolution count value, BR_low_res, corresponds to how many counts of the low-resolution counter (604) occur during a base pacing rate interval; and
wherein the calculated high-resolution count value, C_high_res, is calculated based on the programmed AV interval; and
wherein the controller (112) is also configured to determine as the compensation offset the expected high-resolution count value, E_high_res, minus the measured high-resolution count value, M_high_res.

6. The LP (102a, 102b) of claim 5, wherein to calculate the calculated high-resolution count value, C_high_res, based on the programmed AV interval:
if the LP (102a, 102b) is configured to be implanted in or on a ventricular chamber or proximate a left bundle branch, LBB, and to perform pacing of the ventricular chamber, then the calculated high-resolution count value, C_high_res, corresponds to the base pacing rate interval minus a programmed AV interval; or
if the LP (102a, 102b) is configured to be implanted in or on an atrial chamber and to perform pacing of the atrial chamber, then the calculated high-resolution count value, C_high_res, corresponds to the programmed AV interval.

7. The LP (102a, 102b) of claim 6, wherein:
the high-resolution counter (606) comprises a modulo-n counter that is configured to count from zero to n-1 and then reset back to zero; and
the controller (112) is configured to determine that the expected high-resolution count value, E_high_res, is equal to a modulo-n operation on the sum of the beginning high-resolution count value, B_high_res, and the calculated high-resolution count value, C_high_res.

8. The LP (102a, 102b) of any one of claims 5 through 7, wherein to time pacing of the first cardiac chamber during the second period of time, based on the compensation offset that the LP (102a, 102b) determined and stored, the controller (112) is configured to:
starting when the controller (112) most recently controlled the pulse generator (116) to deliver pacing stimulation to the first cardiac chamber, serially one after another in any order, use the low-resolution counter (604) to count to a calculated low-resolution count value, C_low_res, use the high-resolution counter (606) to count to the compensation offset, and use the low-resolution counter (604) to count to the remaining low-resolution count value, R_low_res,
wherein the calculated low-resolution count value, C_low_res, is equal to the measured low-resolution count value, M_low_res, minus the beginning low-resolution count value, B_low_res; and
wherein use of the low-resolution counter (604) to count to the measured low-resolution count value, M_low_res, and to count to the remaining low-resolution count value, R_low_res, is optionally performed by using the low-resolution counter (604) to count to a sum of the measured low-resolution count value, M_low_res, and the remaining low-resolution count value, R_low_res;
and
control the pulse generator (116) to deliver further pacing stimulation to the first cardiac chamber, when the low-resolution counter (604) has finished counting to the calculated low-resolution count value, C_low_res, and to the remaining low-resolution count value, R_low_res, or the sum thereof, and the high-resolution counter (606) has finished counting to the compensation offset, serially one after another in any order.

9. The LP (102a, 102b) of any one of claims 1 through 8, wherein the controller (112) is configured to:
determine a plurality of the compensation offsets based on the count values determined during the first period of time;
determine an average of the plurality of compensation offsets; and
use the average as the compensation offset during the second period of time to compensate for the drift between the timing circuitry (160) of the LP (102a, 102b) and the timing circuitry (160) of the second LP (102b, 102a) so that the AV synchrony is maintained during the second period of time.

10. The LP (102a, 102b) of any one of claims 1 through 9, wherein:
the LP (102b) is configured to be implanted in or on a ventricular chamber or proximate a left bundle branch, LBB, and to perform pacing of the ventricular chamber; and
the second LP (102a) is configured to be implanted in or on an atrial chamber and to perform pacing of the atrial chamber.

11. The LP (102b) of claim 10, wherein to time its pacing of the first cardiac chamber during the second period of time, based on the compensation offset determined and stored during the first period of time, the controller (112) is configured to:
determine an expected time at which the second LP (102a) delivers pacing stimulation to the second cardiac chamber; and
at a compensated AV interval, following the expected time at which the second LP (102a) delivers pacing stimulation to the second cardiac chamber, control the pulse generator (116) to deliver pacing stimulation to the first cardiac chamber;
wherein the compensated AV interval is based on the compensation offset and a programmed AV interval.

12. The LP (102a, 102b) of any one of claims 1 through 9, wherein:
the LP (102a) is configured to be implanted in or on an atrial chamber and to perform pacing of the atrial chamber; and
the second LP (102b) is configured to be implanted in or on a ventricular chamber or proximate a left bundle branch, LBB, and to perform pacing of the ventricular chamber.

13. The LP (102a) of claim 12, wherein to time its pacing of the first cardiac chamber during the second period of time, based on the compensation offset determined and stored during the first period of time, the controller (112) is configured to:
determine an expected time at which the second LP (102b) delivers pacing stimulation to the second cardiac chamber; and
at a compensated ventricular-atrial, VA, interval, following the expected time at which the second LP (102b) delivers pacing stimulation to the second cardiac chamber, control the pulse generator (116) to deliver pacing stimulation to the first cardiac chamber;
wherein the compensated VA interval is based on the compensation offset and a programmed VA interval.

14. The LP (102a, 102b) of any one of claims 1 through 13, wherein during the first period of time, the LP (102a, 102b) is configured to perform, collectively with the second LP (102b, 102a), pacing in accordance with a DOO mode.

15. The LP (102a, 102b) of any one of claims 1 through 14, where during the second period of time the receiver (120, 122) does not receive event messages from the second LP (102b, 102a) for at least one of the following reasons:
extrinsic interference prevents the LP (102a, 102b) from successfully receiving the event messages from the second LP (102b, 102a);
the second LP (102b, 102a) abstains from transmitting the event messages to conserve energy;
the LP (102a, 102b) disables at least a portion of the receiver (120, 122) to conserve energy; or
a communication channel between the first LP (102a, 102b) and the second LP (102b, 102a) is unstable.

16. A multi-chamber leadless pacemaker system comprising:
the LP (102a, 102b) of any one of claims 1 through 15; and
the second LP (102b, 102a).
